# EUROPEAN PATENT SPECIFICATION

(11) **EP 1 084 109 B1**
(45) Date of publication and mention of the grant of the patent: **26.01.2005**
(21) Application number: 99915990.8
(22) Date of filing: 07.05.1999
(51) Int. Cl.: C07D 213/73, A61K 31/44, C07D 401/04, C07D 213/75, C07D 401/12, C07D 471/08, C07D 471/10

(54) **2-AMINOPYRIDINES CONTAINING FUSED RING SUBSTITUENTS AS NITRIC OXIDE SYNTHASE INHIBITORS**
2-AMINOPYRIDINE MIT KONDENSIERTEN RINGSUBSTITUENTEN ALS STICKSTOFF-OXID-SYNTHASE INHIBITOREN
2-AMINOPYRIDINES CONTENANT DES SUBSTITUANTS A ANNEAUX CONDENSES ET CONVENANT COMME INHIBITEURS DE SYNTHASE DE L'OXYDE NITRIQUE

(30) Priority: 03.06.1998 US 87881 P
(43) Date of publication of application: 21.03.2001
(73) Proprietor: Pfizer Products Inc., Groton, Connecticut 06340 (US)
(72) Inventor: LOWE, John, Adams, III, Stonington, CT 06378 (US)
(74) Representative: Wood, David John
(86) International application number: PCT/IB1999/000825
(87) International publication number: WO 1999/062883

(56) References cited:
- WO-A-97/36871
- WO-A-98/34919
- WO-A-99/10339

## Description

The present invention relates to certain 2-aminopyridines containing fused ring substituents that exhibit activity as nitric oxide synthase (NOS) inhibitors, to pharmaceutical compositions containing them and to their use in the treatment of central nervous system disorders, inflammatory disorders, septic shock and other disorders, in mammals (e.g., humans and companion animals).

There are three known isoforms of NOS - an inducible form (I-NOS) and two constitutive forms referred to as, respectively, neuronal NOS (N-NOS) and endothelial NOS (E-NOS). Each of these enzymes carries out the conversion of arginine to citrulline while producing a molecule of nitric oxide (NO) in response to various stimuli. It is believed that excess nitric oxide (NO) production by NOS plays a role in the pathology of a number of disorders and conditions in mammals. For example, NO produced by I-NOS is thought to play a role in diseases that involve systemic hypotension such as toxic shock and therapy with certain cytokines. It has been shown that cancer patients treated with cytokines such as interleukin 1 (IL-1), interleukin 2 (IL-2) or tumor necrosis factor (TNF) suffer cytokine-induced shock and hypotension due to NO produced from macrophages, i.e., inducible NOS (I-NOS), see Chemical & Engineering News, Dec. 20, p. 33, (1993). I-NOS inhibitors can reverse this. It is also believed that I-NOS plays a role in the pathology of diseases of the central nervous system such as ischemia. For example, inhibition of I-NOS has been shown to ameliorate cerebral ischemic damage in rats, see Am. J. Physiol., **268,** p. R286 (1995)). Suppression of adjuvant induced arthritis by selective inhibition of I-NOS is reported in Eur. J. Pharmacol.. **273,** p. 15-24 (1995).

NO produced by N-NOS is thought to play a role in diseases such as cerebral ischemia, pain, and opiate tolerance. For example, inhibition of N-NOS decreases infarct volume after proximal middle cerebral artery occlusion in the rat, see J. Cerebr. Blood Flow Metab., **14** p. 924-929 (1994). N-NOS inhibition has also been shown to be effective in antinociception, as evidenced by activity in the late phase of the formalin-induced hindpaw licking and acetic acid-induced abdominal constriction assays, see Br. J. Pharmacol., **110,** p. 219-224 (1993). In addition, subcutaneous injection of Freund's adjuvant in the rat induces an increase in NOS-positive neurons in the spinal cord that is manifested in increased sensitivity to pain, which can be treated with NOS inhibitors, see Japanese Journal of Pharmacology, **75,** p. 327-335 (1997). Finally, opioid withdrawal in rodents has been reported to be reduced by N-NOS inhibition, see Neuropsychopharmacol., **13,** p. 269-293 (1995).

WO-A-97/36871 describes certain 6-phenylpyridyl-2-amine derivatives as NOS inhibitors.

### Summary of the Invention

The present invention relates to compounds of the formula 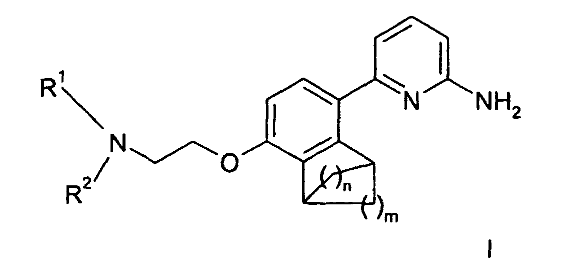 wherein n and m in the bridging rings are independently 1, 2 or 3, and a carbon in one of said bridging rings may be substituted by a heteroatom selected from O, S and N, with the proviso that a bridgehead carbon can only be substituted by nitrogen, and R¹ and R² are independently selected from C₁ to C₆ alkyl, which may be linear, branched or cyclic, and wherein each of R¹ and R² may be independently optionally substituted with from one to three substituents, preferably from zero to two substituents, that are selected, independently, from halo (e.g., chloro, fluoro, bromo iodo), nitro, hydroxy, cyano, amino, (C₁-C₄) alkoxy, and (C₁-C₄) alkylamino;
or R¹ and R² form, together with the nitrogen to which they are attached, a piperazine, azetidine, piperidine or pyrrolidine ring or an azabicyclic ring containing from 6 to 14 ring members, from 1 to 3 of which are nitrogen and the rest of which are carbon,or an azabicyclic ring of the formula 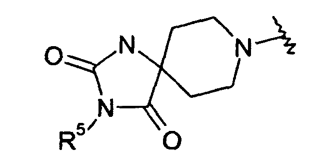 wherein R⁵ is selected from hydrogen, C₁ to C₆ alkyl, phenyl, naphthyt, phenyl-C₁ to C₆ alkyl- and naphthyl C₁ to C₆ alkyl-;
wherein the distal nitrogen on said piperazine or azabicylic ring is optionally substituted with groups R³ and R⁴ wherein R³ and R⁴ are selected from hydrogen, C₁ to C₆ alkyl, phenyl, naphthyl, C₁ to C₆ alkyl-C(=O)-, HC(=O)-, C₁ to C₆ alkoxy-(C=O)-. phenyl-C(=O)-, naphthyl-C(=O)-, and R⁶R⁷NC(=O)- wherein R⁶ and R⁷ are selected, independently, from hydrogen and C₁ to C₆ alkyl, with the proviso that when said azabicyclic ring is a spirocyclic ring, the distal nitrogen on said spirocyclic ring is optionally substituted with R⁵ wherein R⁵ is selected from hydrogen, C₁ to C₆ alkyl, phenyl, naphthyl, phenyl-C₁ to C₆ alkyl- and naphthyl C₁ to C₆ alkyl-;
and wherein said piperazine, azetidine, piperidine and pyrrolidine rings may optionally be substituted with one or more substituents, preferably with from zero to two substituents that are selected, independently, from C₁ to C₆ alkyl, amino, C₁ to C₆ alkylamino, [di-C₁-C₆ alkyl]amino, phenyl substituted 5 to 6 membered heterocyclic rings containing from 1 to 4 rings nitrogen atoms, benzoyl, benzoylmethyl, benzytcarbonyl, phenylaminocarbonyl, phenylethyl and phenoxycarbonyl, and wherein the phenyl moieties of any of the foregoing substituents may optionally be substituted with one or more substituents, preferably with from zero to two substituents, that are selected, independently, from halo, C₁ to C₃ alkyl, C₁ to C₃ alkoxy, nitro, amino, cyano, CF₃ and OCF₃;
with the proviso that no carbon atom is substitued with more than one subsitutent selected from hydroxy, amino, alkoxy, alkylamino and dialkylamino;
and the pahrmaceutically acceptable salts of said compounds.

Examples of the azabicyclic rings that may be formed by NR¹R² are 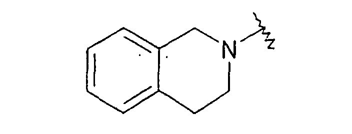 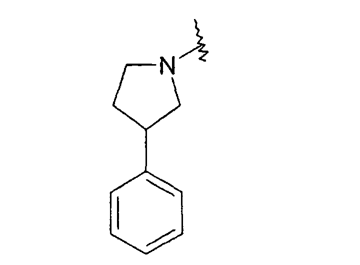 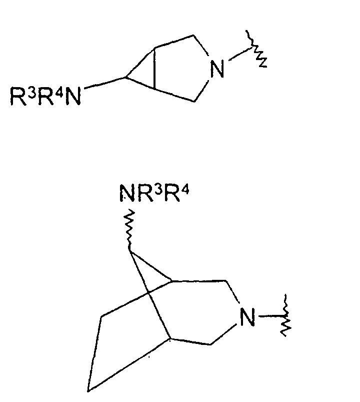 and 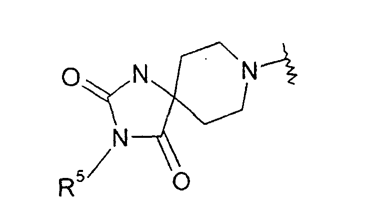 wherein R³ and R⁴ are selected from hydrogen, C₁ to C₆ alkyl, phenyl, naphthyl, C₁ to C₆ alkyl-C(=O)-, HC(=O)-. C₁ to C₆ alkoxy-(C=O)-, phenyl-C(=O)-, naphthyl-C(=O)-, and R⁶R⁷NC(=O)- wherein R⁶ and R⁷ are selected, independently, from hydrogen and C₁ to C₆ alkyl; and R⁵ is selected from hydrogen, C₁ to C₅ alkyl, phenyl, naphthyl, phenyl-C₁ to C₆ alkyl- and naphthyl C₁ to C₆ alkyl-.

A preferred embodiment of the present invention relates to a compound of formula I wherein NR¹R² is an optionally substituted piperidine. azetidine, piperazine or pyrrolidine ring or a 3-aza-bicyclo[3.1.0]hex-6-ylamine ring;
and wherein said piperazine, azetidine, piperidine, pyrrolidine and 3-aza-bicyclo[3.1.0]hex-6-ylamine rings may optionally be substituted with one or more substituents. preferably with from zero to two substituents that are selected, independently, from C₁ to C₆ alkyl, amino, C₁ to C₆ alkylamino, [di-C₁ to C₆ alkyl]amino, phenyl, substituted 5 to 6 membered heterocyclic rings containing from 1 to 4 rings nitrogen atoms, benzoyl. benzoylmethyl, benzylcarbonyl, phenylaminocarbonyl, phenylethyl and phenoxycarbonyl, and wherein the phenyl moieties of any of the foregoing substituents may optionally be substituted with one or more substituents, preferably with from zero to two substituents, that are selected, independently, from halo, C₁ to C₃ alkyl, C₁ to C₃ alkoxy, nitro, amino, cyano. CF₃ and OCF₃;
and the pharmaceutically acceptable salts of said compound.

The following compounds are preferred compounds of the invention:
6-[8-(2-Dimethylamino-ethoxy)-1,2,3,4-tetrahydro-1,4-methano-naphthalen-5-yl]-pyridin-2-ylamine;
6-[8-(2-Pyrrolidin-1-yl-ethoxy)-1,2,3,4-tetrahydro-1,4-methano-naphthalen-5-yl]-pyridin-2-ylamine;
8-[8-(2-Dimethylamino-ethoxy)-1,2,3,4-tetrahydro-1,4-ethano-naphthalen-5-yl]-pyridin-2-ylamine;
6-[8-(2-Pyrrolidin-1-yl-ethoxy)-1,2,3,4-tetrahydro-1,4-ethano-naphthalen-5-yl]-pyridin-2-ylamine;
6-[8-(4-methylpiperazin-1-yl-ethoxy)-1,2,3,4-tetrahydro-1,4-ethano-naphthalen-5-yl]-pyridin-2-ylamine; and
6-[8-(4-(2-phenethyl)-piperazin-1-y1-ethoxy)-1,2.3,4-tetrahydro-1.4-ethano-naphthalen-5-yl]pyridin-2-ylamine.

Other compounds of the invention include the following:
6-[8-(2-(4-Dimethylamino-piperidin-1-yl)-ethoxy)-1,2,3,4-tetrahydro-1,4-methano-naphthalen-5-yl]-pyridin-2-ylamine;
6-[8-(2-(6,7-Dimethoxy-tetrahydroisoquinol-2-yl)-ethoxy)-1,2,3,4-tetrahydro-1,4-methano-naphthalen-5-yl]-pyridin-2-ylamine: and
6-[8-(2-(4-Methylpiperazin-1-yl)-ethoxy)-1,2,3,4-tetrahydro-1,4-methano-naphthalen-5-yl]-pyridin-2-ylamine.

The present invention also relates to the pharmaceutically acceptable acid addition salts of compounds of the formula I. The acids which are used to prepare the pharmaceutically acceptable acid addition salts of the aforementioned base compounds of this invention are those which form non-toxic acid addition salts, i.e., salts containing pharmacologically acceptable anions, such as the hydrochloride, hydrobromide, hydroiodide, nitrate, sulfate, bisulfate, phosphate, acid phosphate, acetate, lactate, citrate, acid citrate, tartrate, bitartrate, succinate, maleate, fumarate, gluconate, saccharate, benzoate, methanesulfonate, ethanesulfonate, benzenesulfonate, p-toluenesulfonate and pamoate [i.e., 1,1-methylene-bis-(2-hydroxy-3-naphthoate)] salts.

The alkyl groups referred to herein and the alkyl portions of other groups referred to herein, unless otherwise indicated, include saturated monovalent hydrocarbon radicals having straight, branched or cyclic moieties or combinations thereof.

The term "one or more substituents", as used herein, refers to a number of substituents that equals from one to the maximum number of substituents possible based on the number of available bonding sites.

The terms "halo" and "halogen", as used herein, unless otherwise indicated. include chloro, fluoro, bromo and iodo.

Examples of more specific embodiments of the present invention include:
(a) compounds of the formula I wherein n is 1;
(b) compounds of the formula I wherein n is 2;
(c) compounds of the formula I wherein m is 1;
(d) compounds of the formula I wherein m is 2;
(e) compounds of the formula I wherein X is oxygen;
(f) compounds of the formula I wherein R¹ and R² are selected. independently, from C₁ to C₆ alkyl;
(g) compounds of the formula I wherein R¹ and R² do not form a ring with the nitrogen to which they are attached;
(h) compounds of the formula I wherein R¹ and R² form, together with the nitrogen to which they are attached, a piperazine, azetidine, piperidine or pyrrolidine ring; and
(i) compounds of the formula I wherein R¹ is selected from C₁ to C₆ alkyl and R² is cyclopropyl.

The present invention also relates to a pharmaceutical composition for treating a condition selected from the group consisting of migraine, inflammatory diseases (e.g., asthma and psoriasis). stroke, acute and chronic pain, hypovolemic shock, traumatic shock, reperfusion injury, Crohn's disease, ulcerative colitis, septic shock, multiple sclerosis, AIDS associated dementia, neurodegenerative diseases (e.g., Parkinson's disease), neuron toxicity, Alzheimer's disease, chemical dependencies and addiction (e.g., dependencies on drugs, alcohol and nicotine), emesis, epilepsy, anxiety, depression, psychosis, head trauma, adult respiratory distress syndrome (ARDS), morphine induced tolerance and withdrawal symptoms, inflammatory bowel disease, osteoarthritis, rheumatoid arthritis, ovulation, dilated cardiomyopathy, acute spinal cord injury, Huntington's disease, ocular diseases (e.g., glaucoma and macular degeneration), diabetic neuropathy, diabetic nephropathy and cancer (e.g., leukemia) in a mammal, including a human, comprising an amount of a compound of the formula I, or a pharmaceutically acceptable salt thereof that is effective in treating or preventing such condition, and a pharmaceutically acceptable carrier.

The present invention also relates to a method of treating a condition selected from the group consisting of migraine, inflammatory diseases (e.g., asthma and psoriasis), stroke, acute and chronic pain, hypovolemic shock, traumatic shock, reperfusion injury, Crohn's disease, ulcerative colitis. septic shock, multiple sclerosis, AIDS associated dementia, neurodegenerative diseases, (e.g., Parkinson's disease), neuron toxicity, Alzheimer's disease, chemical dependencies and addictions (e.g., dependencies on drugs, alcohol and nicotine), emesis, epilepsy, anxiety, depression, psychosis, head trauma, adult respiratory distress syndrome (ARDS), morphine induced tolerance and withdrawal symptoms, inflammatory bowel disease, osteoarthritis, rheumatoid arthritis, ovulation, dilated cardiomyopathy, acute spinal cord injury, Huntington's disease, ocular diseases (e.g., glaucoma and macular degeneration), diabetic neuropathy, diabetic nephropathy and cancer (e.g., leukemia) in a mammal, including a human, comprising administering to said mammal an amount of a compound of the formula I, or a pharmaceutically acceptable salt thereof, that is effective in treating or preventing such condition.

The present invention also relates to a pharmaceutical composition for inhibiting nitric oxide synthase (NOS) in a mammal. including a human, comprising an NOS inhibiting effective amount of a compound of the formula I, or a pharmaceutically acceptable salt thereof and a pharmaceutically acceptable carrier.

The present invention also relates to a method of inhibiting NOS in a mammal, including a human, comprising administering to said mammal a NOS inhibiting effective amount of a compound of the formula I, or a pharmaceutically acceptable salt thereof.

The present invention also relates to a pharmaceutical composition for treating a condition selected from the group consisting of migraine, inflammatory diseases (e.g., asthma and psoriasis), stroke, acute and chronic pain, hypovolemic shock, traumatic shock, reperfusion injury, Crohn's disease, ulcerative colitis, septic shock, multiple sclerosis, AIDS associated dementia, neurodegenerative diseases (e.g., Parkinson's disease), neuron toxicity, Alzheimer's disease, chemical dependencies and addictions (e.g., dependencies on drugs, alcohol and nicotine), emesis, epilepsy, anxiety, depression, psychosis, head trauma, adult respiratory distress syndrome (ARDS), morphine induced tolerance and withdrawal symptoms, inflammatory bowel disease, osteoarthritis, rheumatoid arthritis, ovulation, dilated cardiomyopathy, acute spinal cord injury, Huntington's disease, ocular diseases (e.g., glaucoma and macular degeneration), diabetic neuropathy, diabetic nephropathy and cancer (e.g., leukemia) in a mammal, including a human, comprising a NOS inhibiting effective amount of a compound of the formula I, or a pharmaceutically acceptable salt thereof and a pharmaceutically acceptable carrier.

The present invention also relates to a method of treating a condition selected from the group consisting of migraine, inflammatory diseases (e.g., asthma and psoriasis), stroke, acute and chronic pain, hypovolemic shock, traumatic shock, reperfusion injury, Crohn's disease, ulcerative colitis, septic shock, multiple sclerosis, AIDS associated dementia, neurodegenerative diseases (e.g., Parkinson's disease), neuron toxicity, Alzheimer's disease, chemical dependencies and addictions (e.g., dependencies on drugs, alcohol or nicotine), emesis, epilepsy, anxiety, depression, psychosis, head trauma, adult respiratory distress syndrome (ARDS), morphine induced tolerance and withdrawal symptoms, inflammatory bowel disease, osteoarthritis, rheumatoid arthritis, ovulation, dilated cardiomyopathy, acute spinal cord injury, Huntington's disease, ocular diseases (e.g., glaucoma and macular degeneration), diabetic neuropathy, diabetic nephropathy and cancer (e.g., leukemia) in a mammal, including a human, comprising administering to said mammal a NOS inhibiting effective amount of a compound of the formula II, or a pharmaceutically acceptable salt thereof.

The present invention also relates to compounds of the formula 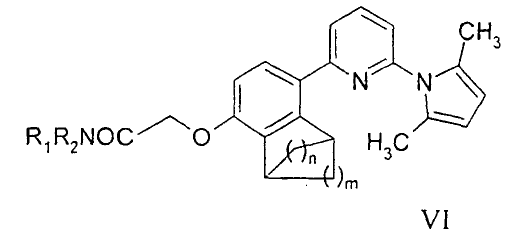 wherein n, m, R¹, and R² are as defined above for compounds of formula I. Compounds of formula VI are useful as intermediates for preparing compounds of formula I.

The present invention also relates to compounds of the formula 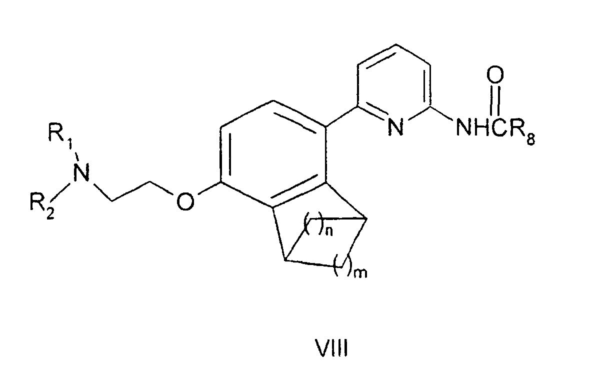 wherein n, m, R¹, and R² are as defined above for compounds of formula I, and wherein R⁸ is aryl, such as phenyl or naphthyl. Compounds of formula VIII are useful as intermediates for preparing compounds of formula I.

As used herein unless indicated otherwise, the term "treating" should be understood as including "preventing".

Compounds of formula I may contain chiral centers and therefore may exist in different enantiomeric and diastereomeric forms. This invention relates to all optical isomers and all stereoisomers of compounds of the formula I and mixtures thereof, and to all pharmaceutical compositions and methods of treatment defined above that contain or employ them, respectively.

Formula I above includes compounds identical to those depicted but for the fact that one or more hydrogen, carbon or other atoms are replaced by isotopes thereof Such compounds may be useful as research and diagnostic tools in metabolism pharmacokinetic studies and in binding assays.

### Detailed Description of the Invention

The compounds of the formula I may be prepared as described in the following reaction schemes and discussion. Unless otherwise indicated, n, m, R¹ and R² in the reaction schemes and discussion that follow are defined as above.

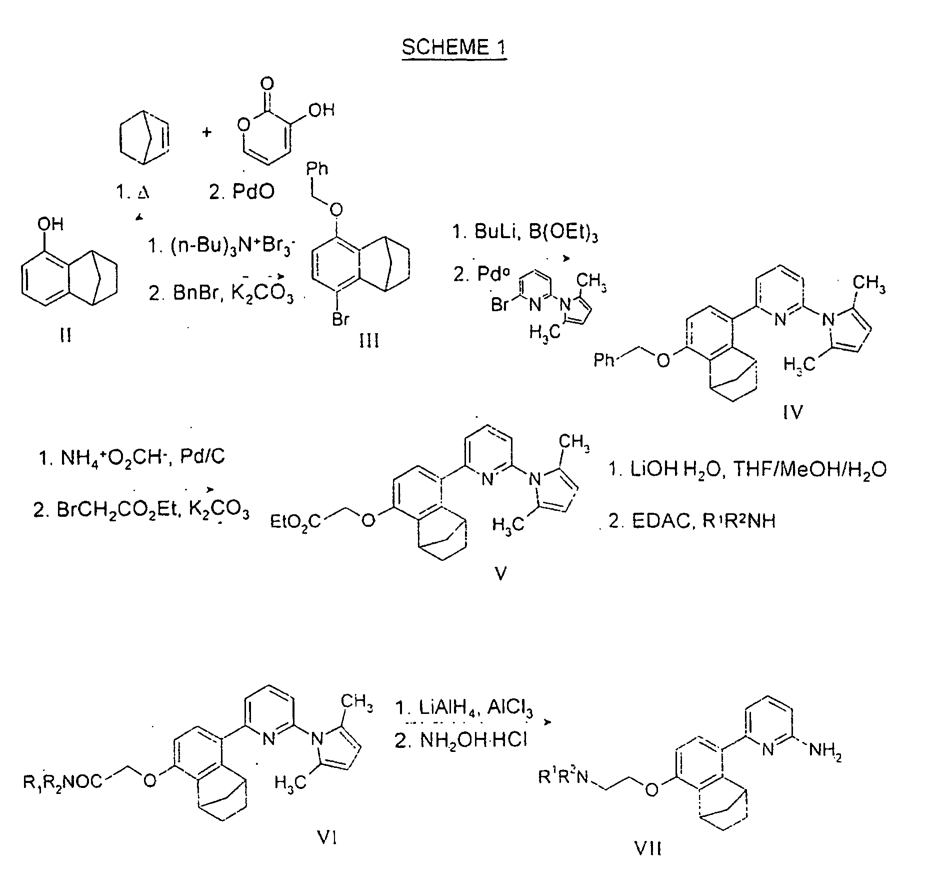

Referring to Scheme 1, the compound of formula II is prepared by reaction of norbornylene and 2-hydroxypyrone followed by aromatization with palladium oxide, according to the procedure described in Syn. Commun.,**5**, 461, (1975). It is then reacted with tetrabutylammonium tribromide in 1,2-dichloroethane at about room temperature for about 10 minutes to about 10 hours. The product of this reaction is then treated with benzyl bromide and potassium carbonate in a solvent such as acetonitrile, at about the reflux temperature of the reaction mixture for about 1 to 48 hours, to form the compound of formula III.

The compound of formula III is then converted into 5-benzyloxy-1,2,3,4-tetrahydro-1,4-methano-naphthalene-8-boronic acid by cooling the compound of formula III to about -70°C in dry tetrahydrofuran (THF), and adding a solution of n-butyl lithium to it. The resulting solution is then treated with triethyl borate and allowed to warm to room temperature for about 1 to 48 hours to form 5-benzyloxy-1,2,3,4-tetrahydro-1,4-methano-naphthalene-8-boronic acid. Reaction of 5-benzyloxy-1,2.3,4-tetrahydro-1,4-methano-naphthalene-8-boronic acid with 6-bromo-2-(2.5-dimethylpyrrolyl)pyridine in an ethanol solvent, in the presence of sodium carbonate and tetrakistriphenylphosphine palladium, at about the reflux temperature for about 1 to 48 hours of the reaction mixture, yields the compound of formula IV.

The compound of formula IV can be converted into the compound of formula V using the following two step process. The compound of formula IV is reacted with ammonium formate and ten percent palladium on carbon, in an ethanol solvent, at about the reflux temperature of the reaction mixture, for about 10 minutes to about 10 hours to yield the analogous compound to that having formula IV, wherein the benzyloxy group of formula IV is replaced with a hydroxy group. The compound of formula V is then formed by reacting the above hydroxy derivative with 2-bromoethylacetate and potassium carbonate in acetonitrile at about the reflux temperature of the reaction mixture for about 1 to 48 hours.

Basic hydrolysis of the compound of formula V, followed by reaction with N-ethyl-N-3-dimethylaminopropylcarbodiimide (EDAC) and the appropriate compound having the formula R¹R²NH yields the desired compound of the formula VI. The base hydrolysis is typically carried out using an alkali metal or alkaline earth metal hydroxide in a mixture of THF, methanol and water at about room temperature for about 1 to 48 hours. The reaction of the compound of formula VI with the appropriate compound of the formula R¹R²NH and N-ethyl-N-dimethylaminopropyl carbodiimide (EDAC) is conducted in the presence of a base. Examples of suitable bases are those selected from trialkylamines, alkali metal carbonates and alkaline earth metal carbonates. This reaction is typically conducted in a solvent such as acetonitrile, methylene chloride or N,N-dimethylformamide (DMF), at a temperature from about room temperature to about 100°C, preferably at about room temperature for about 1 to 48 hours. Preferably, the reaction is conducted in the presence of a catalytic additive such as N-hydroxysuccinamide or hydroxybenzotriazole

The compound of formula VI can be converted into the desired compound of formula I as follows. The compound of formula VI is reduced to form the corresponding compound wherein the carbonyl group is replaced by a methylene group, namely 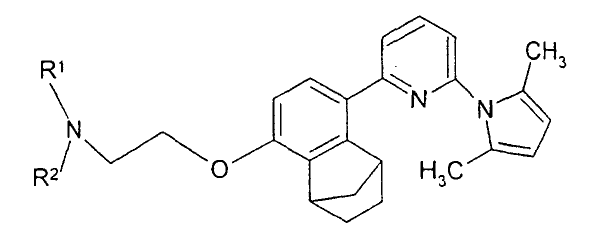 after which the 2.5-dimethylpyrrolyl protecting group is removed. The reduction can be carried out using methods well known to those of skill in the art, for example, using lithium aluminum hydride in tetrahydrofuran, with or without aluminum chloride, or using borane methyl sulfide in tetrahydrofuran, at a temperature of about -78°C to about reflux. preferably at about -70°C to room temperature for about 1 to about 24 hours.

Removal of the 2,5-dimethylpyrrolyl protecting group can be accomplished by reaction with hydroxylamine hydrochloride. This reaction is generally carried out in an alcoholic or aqueous alcoholic solvent (preferably, using ethanol as the alcohol), at a temperature from about room temperature to about the reflux temperature of the reaction mixture, preferably at about the reflux temperature, for about 8 to about 72 hours.

Compounds of the formula I that are identical to those of formula VII but for the fact that there is a heteroatom in one of the bridging rings can be prepared in an analogous fashion to that depicted in Scheme 1, starting with the appropriate compound that is analogous to that of formula II, wherein the unsubstituted bridged ring of formula II is replaced by a bridged ring comprising a heteroatom.

A compound of formula I that is identical to that of formula VII, except for that each bridging ring comprises more or less atoms (as permitted by formula I), can be prepared in an analogous fashion to that depicted in Scheme 1, starting with an analog of formula II having bridging rings containing the appropriate number of atoms.

An anaolgous compound to formula II can be prepared as depicted in Scheme 1 and described above, starting with a compound having the formula

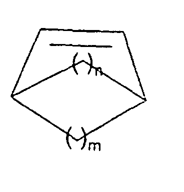

Compounds of formula I wherein n is 2 and m is 1 can also be prepared using 5-methoxy-1,4-dihydro-1,4-ethano-naphthalene (J. Med. Chem., **30**, 2191 (1987)) as a starting material. The 5-methoxy-1,4-dihydro-1,4-ethano-naphthalene can be converted into 5-hydroxy-1,2,3,4-tetrahydro-1,4-ethano naphthalene (the n=2 analog of the compound of formula II of Scheme 1) by hydrogenation followed by demethylation. Compounds of formula 1 wherein n is 2 and m is 1 can then be prepared from 5-hydroxy-1,2,3,4-tetrahydro-1,4-ethano naphthalene following the remainder of Scheme 1, subsequent to the synthesis of the compound of formula II.

Compounds of formula I can also be prepared from compounds of formula VIII, as depicted in the following Scheme 2:

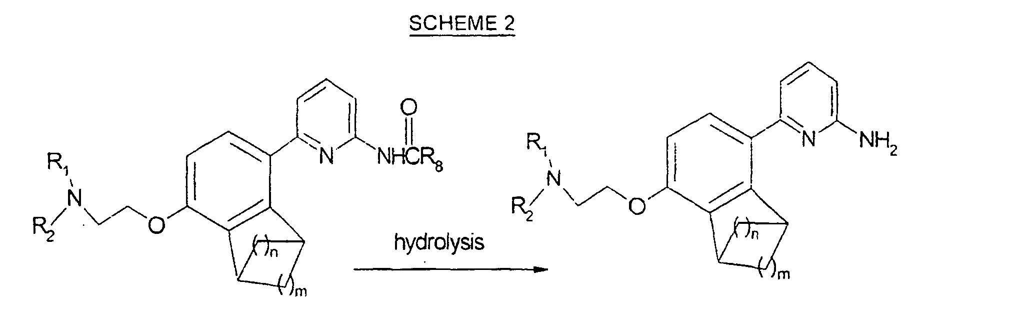

Referring to Scheme 2, R⁸ is selected from the group consisting of aryl and C₁ to C₆ alkyl. wherein aryl is preferably phenyl or naphthyl. A compound of formula I is obtained by hydrolysis of a compound of formula VIII with an acid or a base. Examples of acids that can be used include, but are not limited to, a mineral acid and sulfonic acid. Examples of bases that can be used include an alkyli hydroxide and an alkyli metal hydroxide. The hydrolysis of the compound of formula VIII can be performed in an alcoholic or aqueous solvent, at a temperature of from about zero to about 100°C, for about 1 to 24 hours.

The preparation of other compounds of the formula I not specifically described in the foregoing experimental section can be accomplished using combinations of the reactions described above that will be apparent to those skilled in the art.

In each of the reactions discussed or illustrated above, pressure is not critical unless otherwise indicated. Pressures from about 0.5 atmospheres to about 5 atmospheres are generally acceptable, and ambient pressure, i.e., about 1 atmosphere, is preferred as a matter of convenience.

The compounds of formulae I ("the active compounds of this invention") which are basic in nature are capable of forming a wide variety of different salts with various inorganic and organic acids. Although such salts must be pharmaceutically acceptable for administration to animals, it is often desirable in practice to initially isolate a compound of the formula I from the reaction mixture as a pharmaceutically. unacceptable salt and then simply convert the latter back to the free base compound by treatment with an alkaline reagent and subsequently convert the latter free base to a pharmaceutically acceptable acid addition salt. The acid addition salts of the active base compounds of this invention are readily prepared by treating the base compound with a substantially equivalent amount of. the chosen mineral or organic acid in an aqueous solvent medium or in a suitable organic solvent, such as methanol or ethanol. Upon careful evaporation of the solvent, the desired solid salt is readily obtained.

The active compounds of this invention and their pharmaceutically acceptable salts are useful as NOS inhibitors i.e., they possess the ability to inhibit the NOS enzyme in mammals, and therefore they are able to function as therapeutic agents in the treatment of the aforementioned disorders and diseases in an afflicted mammal.

The active compounds of this invention and their pharmaceutically acceptable salts can be administered via either the oral, parenteral or topical routes. In general, these compounds are most desirably administered in dosages ranging from about 0.01 to about 250 mg per day, in single or divided doses (i.e., from 1 to 4 doses per day), although variations will necessarily occur depending upon the species, weight and condition of the subject being treated and the particular route of administration chosen. However, a dosage level that is in the range of about 0.07 mg to about 21 mg per kg of body weight per day is most desirably employed. Variations may nevertheless occur depending upon the species of animal being treated and its individual response to said medicament, as well as on the type of pharmaceutical formulation chosen and the time period and interval at which such administration is carried out. In some instances, dosage levels below the lower limit of the aforesaid range may be more than adequate, while in other cases still larger doses may be employed without causing any harmful side effect, provided that such larger doses are first divided into several small doses for administration throughout the day.

The active compounds of the invention may be administered alone or in combination with pharmaceutically acceptable carriers or diluents by either of the three routes previously indicated, and such administration may be carried out in single or multiple doses. More particularly, the novel therapeutic agents of this invention can be administered in a wide variety of different dosage forms, i.e., they may be combined with various pharmaceutically acceptable inert carriers in the form of tablets, capsules, lozenges, troches, hard candies, powders, sprays, creams, salves, suppositories, jellies, gels, pastes, lotions, ointments, aqueous suspensions, injectable solutions, elixirs, syrups, and the like. Such carriers include solid diluents or fillers, sterile aqueous media and various non-toxic organic solvents, etc. Moreover, oral pharmaceutical compositions can be suitably sweetened and/or flavored. In general, the therapeutically-effective compounds of this invention are present in such dosage forms at concentration levels ranging from about 5.0% to about 70% by weight.

For oral administration, tablets containing various excipients such as microcrystalline cellulose, sodium citrate, calcium carbonate, dicalcium phosphate and glycine may be employed along with various disintegrants such as starch (and preferably corn, potato or tapioca starch), alginic acid and certain complex silicates, together with granulation binders like polyvinylpyrrolidone, sucrose, gelatin and acacia. Additionally, lubricating agents such as magnesium stearate, sodium lauryl sulfate and talc are often very useful for tabletting purposes. Solid compositions of a similar type may also be employed as fillers in gelatin capsules; preferred materials in this connection also include lactose or milk sugar as well as high molecular weight polyethylene glycols. When aqueous suspensions and/or elixirs are desired for oral administration, the active ingredient may be combined with various sweetening or flavoring agents, coloring matter or dyes, and, if so desired, emulsifying and/or suspending agents as well, together with such diluents as water, ethanol, propylene glycol, glycerin and various like combinations thereof.

For parenteral administration, solutions of an active compound of the present invention in either sesame or peanut oil or in aqueous propylene glycol may be employed. The aqueous solutions should be suitably buffered (preferably pH greater than 8) if necessary and the liquid diluent first rendered isotonic. These aqueous solutions are suitable for intravenous injection purposes. The oily solutions are suitable for intraarticular, intramuscular and subcutaneous injection purposes. The preparation of all these solutions under sterile conditions is readily accomplished by standard pharmaceutical techniques well known to those skilled in the art.

Additionally, it is also possible to administer the active compounds of the present invention topically when treating inflammatory conditions of the skin and this may be done by way of creams, jellies, gels, pastes, patches, ointments and the like, in accordance with standard pharmaceutical practice.

The ability of compounds of the formulae I to inhibit NOS may be determined using procedures described in the literature. The ability of compounds of the formulae I to inhibit endothelial NOS may be determined by using the procedures described by Schmidt et al. in Proc. Natl. Acad. Sci. U.S.A., 88, pp. 365-369 (1991) and by Pollock et al., in Proc. Natl. Acad. Sci. U.S.A., 88, pp. 10480-10484 (1991). The ability of compounds of the formulae I to inhibit inducible NOS may be determined using the procedures described by Schmidt et al., in Proc. Natl. Acad. Sci. U.S.A., 88 pp. 365-369 (1991) and by Garvey et al. in J. Biol. Chem., 269, pp. 26669-26676 (1994). The ability of the compounds of the formulae I to inhibit neuronal NOS may be determined using the procedure described by Bredt and Snyder in Proc. Natl. Acad. Sci. U.S.A., 87, 682-685 (1990). Of the two compounds of the formula that were tested, both exhibited an IC₅₀ < 10 µM for inhibition of either inducible or neuronal NOS.

The present invention is illustrated by the following examples. It will be understood, however, that the invention is not limited to the specific details of these examples. Melting points are uncorrected. Proton nuclear magnetic resonance spectra (¹H NMR) and ¹³C nuclear magnetic resonance spectra were measured for solutions in deuterochloroform (CDCl₃) or in CD₃OD or CD₃SOCD₃ and peak positions are expressed in. parts per million (ppm) downfield from tetramethylsilane (TMS). The peak shapes are denoted as follows: s, singlet; d, doublet; t, triplet: q, quartet, m, multiplet, b, broad.

### EXAMPLE 1

### 6-[8-(2-Dimethylamino-ethoxy)-1,2,3,4-tetrahydro-1,4-methano-naphthalen-5-yl]-pyridin-2-ylamine:

### A. 5-Hydroxy-1,2,3,4-tetrahydro-1,4-methano-naphthalene

Norbornylene (18.1 g, 190 mmol) was reacted with the known (Syn. Commun., **5**, 461, (1975)) 2-hydroxypyrone (5.38 g, 48 mmol) neat in a sealed tube at 125°C for 2 days to give a mixture of 1,2- and 1,4- 5,6,7,8-octahydro-5,8-methano-naphth-1-one in 45% and 4% yields respectively, which was converted to the desired phenol by refluxing 48 hours with palladium oxide (0.5 g) and magnesium sulfate (1.0 g) in toluene (80 mL), in 71% yield.
¹H-NMR (δ, CDCl₃): 1.20 (m, 2H), 1.50 (m, 1H), 1.75 (m, 1H), 1.90 (m, 2H). 3.355 (m, 1H), 3.55 (m, 1H), 5.28 (bs, 1H), 6.59 (d, J=8, 1H), 6.79 (d, J=7, 1H), 6.95 (t, J=8, 1H).
¹³C-NMR (δ, CDCl₃): 26.4, 27.0, 39.2, 43.9, 49.0, 113.1, 113.4, 126.7, 132.4, 148.6, 150.65.
MS (%): 160 (parent, 100).

### B. 5-Hydroxy-8-bromo-1,2,3,4-tetrahydro-1,4-methano-naphthalene

To a 250 mL round-bottomed flask equipped with addition funnel and N₂ inlet were added 2.9 g (18 mmol) 5,6,7,8-tetrahydro-1,4-methano-naphthalen-1-ol and 50 mL 1,2-dichloroethane, and with stirring a solution of 8.7 g (18 mmol) tributylammonium tribromide in 30 mL 1,2-dichloroethane dropwise over 10 minutes. After stirring an additional 10 minutes at room temperature, the solution was washed with water, dilute aqueous sodium bisulfite, and water, dried over sodium sulfate, and evaporated. The mixture of product and tributylammonium bromide was chromatographed on silica gel using hexane/ethyl acetate as eluant to afford an oil, 3.2 g (74%).
¹H-NMR (δ, CDCl₃): 1.20 (m, 2H), 1.50 (m, 1H), 1.75 (m, 1H), 1.90 (m, 2H), 3.53 (m, 1H), 3.58 (m, 1H), 4.80 (bs, 1H), 6.46 (d, J=9, 1H), 7.04 (d, J=9, 1H).

### C. 5-Benzyloxy-8-bromo-1,2,3,4-tetrahydro-1,4-methano-naphthalene

The above oil was dissolved in 50 mL acetonitrile, and treated with 1.7 mL (14.4 mmol) benzyl bromide and 3.6 g (26.4 mmol) potassium carbonate, then refluxed 14 hours. TLC showed a major spot at R_{f} = 0.3 in 10% methylene chloride/hexane (with benzyl bromide at R_{f}=0.4). The reaction was cooled, poured into dilute aqueous hydrochloric acid/ethyl acetate, and the organic layer separated, washed with water and brine, dried over sodium sulfate, and evaporated. The residue was chromatographed on silica gel using methylene chloride/hexane as eluant to afford 4.1 g (94%) of an oil.
¹H-NMR (δ, CDCl₃): 1.21 (m, 2H), 1.50 (m, 1H), 1.76 (m, 1H), 1.940 (m, 2H), 3.57 (m. 1H), 3.77.(m, 1H), 5.08 (s, 2H), 6.61 (d, J=9, 1H), 7.14 (d, J=9. 1H), 7.3-7.5 (m, 5H).
¹³C-NMR (δ, CDCl₃): 26.0, 26.2, 41.0. 44.7. 48.5, 70.6, 107.6, 112.6, 127.3. 127.9, 128.8, 129.1, 137.2, 137.6, 149.5, 151.5.
MS (%): 327/327 (parent, Br⁷⁹/Br⁸¹, 100).

### D. 5-Benzyloxy-1,2,3,4-tetrahydro-1,4-methano-naphthalene-8-boronic acid

To a 125 mL round-bottomed flask equipped with N₂ inlet were added 4.1 g (12.5 mmol) 5-benzyloxy-8-bromo-1,2,3,4-tetrahydro-1,4-methano-naphthalene and 20 mL dry tetrahydrofuran. The solution was cooled to -70°C, and 6.5 mL (16.2 mmol) of a 2.5M solution of n-butyl lithium in hexane was added over 5 minutes, and the reaction stirred at -70°C for 10 minutes. The solution was then treated with 2.8 mL (16.2 mmol) triethyl borate, stirred 5 minutes at -70°C, then warmed to room temperature and stirred 40 h. The reaction was quenched with aqueous ammonium chloride solution, poured into 0.5 N hydrochloric acid, and extracted into ethyl acetate. The organic layer was washed with brine, dried over sodium sulfate, and evaporated to a white foam after trituration with hexane. 3.6 g (100%).
¹H-NMR (δ, CDCl₃): 1.2-1.4 (m, 2H), 1.55 (m, 1H), 1.84 (m, 1H), 1.92 (m, 2H), 3.72 (m, 2H), 5.15 and 5.17 (singlets, 2H for the mono-and diaryl boronic acid), 6.77 and 6.83 (doublets, J=8, 1H), 7.2-7.5 (m, 5H), 7.8 and 7.92 (doublets, J=9, 1H).

### E. 2-(2,5-Dimethylpyrrolyl)-6-[8-benzyloxy-1,2,3,4-tetrahydro-1,4-methano-naphthalen-5-yl]-pyridine

To a 500 mL round-bottomed flask equipped with condenser and N₂ inlet were added 3.1 mg (12.2 mmol) 2-(2,5-dimethylpyrrolyl)-6-bromo-pyridine, 3.2 mg (12.2 mmol) 5-benzyloxy-1,2,3,4-tetrahydro-1,4-methano-naphthalene-8-boronic acid, 5.2 g (48.8 mmol) sodium carbonate, 282 mg (0.24 mmol) tetrakistriphenylphosphine palladium, 135 mL ethanol, and 15 mL water, and the reaction heated at 80°C for 13 hours. TLC showed a major spot at R_{f} = 0.4 in 20% ethyl acetate in hexane, and LCMS showed a major peak at P+1 = 421. The reaction was cooled, poured into water, and extracted into ethyl acetate. The organic layer was washed with water and brine, dried over sodium sulfate, and evaporated. The residue was chromatographed on silica gel using hexane/ethyl acetate as eluant to afford 4.8 g (94%) of a white solid.
¹H-NMR (δ, CDCl₃): 1.33 (m, 2H), 1.50 (m, 1H), 1.75 (m, 1H). 1.98 (m. 2H), 2.22 (s, 6H), 3.73 (bs, 1H). 3.85 (bs, 1H), 5.15 (s, 2H), 5.92 (s, 2H), 6.81 (d, J=8.5, 1H), 7.10 (d, J=7.5, 1H), 7.2-7.5 (m, 7H), 7.845 (t, J=8, 1H).
¹³C-NMR (δ, CDCl₃): 13.5, 26.4, 26.7, 39.7, 43.1, 48.9, 70.1, 106.7, 107.6, 110.65, 119.0, 120.3, 121.0, 126.3, 126.4, 127.3, 127.5, 127.8, 128.5. 128.7, 136.3, 137.4, 138.0, 148.4, 151.6, 152.7. 158.0.
MS (%): 421 (parent+1, 100).

### F. 2-(2,5-Dimethylpyrrolyl)-6-[8-hydroxy-1,2,3,4-tetrahydro-1,4-methano-naphthalen-5-yl]-pyridine

To a 250 mL round-bottomed flask equipped with condenser and N₂ inlet were added 4.8 g (11.4 mmol) 2-(2,5-dimethylpyrrolyl)-6-[8-benzyloxy-1,2,3,4-tetrahydro-1.4-methano-naphthalen-5-yl]-pyridine, 3.0 g (47.6 mmol) ammonium formate, 130 mg 10% palladium-on-carbon, and 100 mL ethanol. The reaction was refluxed 4 hours, with additional catalyst and formate added at 2 and 3 hours, then cooled and filtered through diatomaceous earth (Celite (trademark)) with ethanol and methylene chloride. The filtrate was evaporated and the residue taken up in ethyl acetate/aqueous sodium bicarbonate solution. The organic layer was washed with brine, dried over sodium sulfate, and evaporated to a light brown solid, 3.9 g (about 100%).
¹H-NMR (δ, CDCl₃): 1.30 (m, 2H), 1.49 (m, 1H), 1.72 (m, 1H), 1.95 (m, 2H), 2.215 (s, 6H), 3.59 (bs, 1H), 3.81 (bs, 1H), 5.93 (s, 2H), 6.59 (d. J=8.5, 1H), 7.11 (d, J=8. 1H), 7.38 (d, J=8.5, 1H), 7.50 (d, J=8, 1H), 7.855 (t, J=8, 1H).
¹³C-NMR (δ, CDCl₃): 13.4, 26.4, 26.6, 3.3, 43.0, 48.9, 106.8, 107.2, 113.9, 119.2, 121.4, 127.6, 128.8, 133.5, 138.3, 148.6, 150.0, 158.4 (one carbon not indicated).
MS (%): 329 (parent+1, 100).

### G. 2-(2,5-Dimethylpyrrolyl)-6-[8-carboethoxymethoxy-1,2,3,4-tetrahydro-1,4-methano-naphthalen-5-yl]-pyridine

To a 250 mL round-bottomed flask equipped with condenser and N₂ inlet were added 3.9 g (11.9 mmol) 2-(2,5-dimethylpyrrolyl)-6-[8-hydroxy-1,2,3,4-tetrahydro-1,4-methano-naphthalen-5-yl]-pyridine, 1.6 mL (14.2 mmol) ethyl bromoacetate, 2.0 g (14.2 mmol) potassium carbonate, and 80 mL acetonitrile. The mixture was refluxed 12 hours, cooled (TLC Rf = 0.4 in 1/3-ethyl acetate/hexane), poured into water, and extracted into ethyl acetate. The organic layer was washed with brine, dried over sodium sulfate, and evaporated. The residue was chromatographed on silica gel using hexane/ethyl acetate as eluant to afford 3.95 g (80%) of an oil.
¹H-NMR (δ, CDCl₃): 1.29 (t, J=7, 3H), 1.30 (m, 2H), 1.49 (m, 1H), 1.75 (m, 1H), 1.97 (m, 2H), 2.20 (s, 6H), 3.73 (bs, 1H). 3.82 (bs, 1H), 4.25 (q, J=7, 2H), 4.67 (s, 2H), 5.89 (s, 2H), 6.63 (d, J=9, 1H), 7.09 (d, J=8, 1H), 7.49 (m, 2H), 7.835 (t, J=8, 1H).
¹³C-NMR (δ, CDCl₃): 13.4, 14.1, 26.25, 26.5, 39.7, 43.0, 48.8, 61.2, 65.8, 106.6, 106.9, 110.1, 119.0, 120.9, 122.0, 127.4, 128.6, 136.4, 137.9, 148.5, 149.1, 151.8, 157.8, 169.0.
MS (%): 415 (parent+1, 100).

### H. 2-(2,5-Dimethylpyrrolyl)-6-[8-carboxymethoxy-1,2,3,4-tetrahydro-1,4-methano-naphthalen-5-yl]-pyridine

To a 125 mL round-bottomed flask equipped with condenser and N₂ inlet were added 3.95 g (9.5 mmol) of 2-(2,5-dimethylpyrrolyl)-6-[8-carboethoxymethoxy-1,2,3,4-tetrahydro-1,4-methano-naphthalen-5-yl]-pyridine. 30 mL tetrahydrofuran, and 1.2 g (28.6 mmol) lithium hydroxide hydrate in 30 mL water, with additional methanol to maintain a solution. The reaction was stirred at room temperature for 12 hours. (LCMS P+1 = 389), poured into dilute aqueous hydrochloric acid, and extracted into ethyl acetate. The organic layer was washed with brine, dried over sodium sulfate, and evaporated to a solid, 2.4 g (65%).
¹H-NMR (δ, CDCl₃/CD₃OD): 1.28 (m, 2H), 1.51 (m, 1H). 1.70 (m, 1H), 1.95 (m, 2H), 2.13 (s, 6H), 3.715 (bs, 1H), 3.76 (bs, 1H), 4.67 (s, 2H), 4.81 (s, 2H), 6.70 (d, J=8.5, 1H), 7.16 (d, J=8, 1H), 7.38 (d, J=8.5, 1H), 7.55 (d, J=8, 1H), 7.95 (t, J=8, 1H).
¹³C-NMR (δ, CDCl₃): 12.3, 25.9. 26.3, 39.6, 42.85, 65.0, 110.0, 119.7, 121.8, 126.5. 127.2, 128.15, 136.0, 138.7, 148.4, 151.6, 152.1, 158.1, 171.4.
MS (%): 389 (parent+1 , 100).

### 1. 2-(2,5-Dimethylpyrrolyl)-6-[8-(N,N-dimethylcarboxamido)methoxy-1.2,3,4-tetrahydro-1,4-methano-naphthalen-5-yl]-pyridine

To a 100 mL round-bottomed flask equipped with condenser and N₂ inlet were added 200 mg (0.52 mmol) 6-(4-carboxymethylnaphthalen-1-yl)-pyridin-2-ylamine, 85 mg (1.04 mmol) N,N-dimethylamine hydrochloride, 397 mg (2.1 mmol) N-ethyl, N-3-dimethylaminopropylcarbodiimide, 381 mg (3.1 mmol) 4-dimethylaminopyridine, and 10 mL dry acetonitrile. The reaction was stirred at room temperature 12 hours (LCMS showed P+1 = 416 and TLC showed R_{f} = 0.2 in 5% methanol/methylene chloride), then evaporated and the residue chromatographed on silica gel using methanol/methylene chloride as eluant to afford the product as a foam, 202 mg (93.5%).
¹H-NMR (δ, CDCl₃): 1.32 (m, 2H), 1.50 (m, 1H), 1.73 (m, 1H). 1.97 (m, 2H), 2.21 (s; 6H), 2.98 (s, 3H), 3.10 (s, 3H), 3.71 (bs, 1H). 3.85 (bs, 1H), 4.74 (s, 2H), 5.90 (s, 2H), 6.76 (d, J=9, 1H), 7.09 (d, J=8, 1H), 7.49 (m, 2H), 7.84 (t, J=8, 1H).
¹³C-NMR (δ, CDCl₃): 13.42, 26.36, 26.54, 35.65, 36.57, 39.61, 42.99, 48.85, 67.75, 106.62, 110.01, 118.99, 120.90, 126.90, 127.52, 128.58, 135.97, 138.00, 148.43, 151.52, 151.78, 157.87, 167.93.
MS (%): 416 (parent+1, 100).

### J. 2-(2,5-Dimethylpyrrolyl)-6-[8-(N,N-dimethylaminoethoxy)- 1,2,3,4-tetrahydro-1.4-methano-naphthalen-5-yl]-pyridine

To a 100 mL round-bottomed flask equipped with condenser and N₂ inlet were added 196 mg (1.5 mmol) aluminum chloride and 10 mL dry tetrahydrofuran. The solution was cooled to 0°C, and 3.40 mL (3.40 mmol) of a 1.0 M solution of lithium aluminum hydride in tetrahydrofuran was added. Stirring was continued at room temperature for 20 minutes, then the solution was cooled to -70°C, and a solution of 202 mg (0.49 mmol) 2-(2,5-dimethylpyrrolyl)-6-[8-(N,N-dimethylcarboxamidomethoxy)-1,2,3,4-tetrahydro-1,4-methano-naphthalen-5-yl]-pyridine in 10 mL dry tetrahydrofuran was added. Stirring was continued for 1 hour at -70°C, then 2 hours at room temperature (LCMS showed P+1 = 402), followed by careful quenching with 5 mL 1 N hydrochloric acid. After being stirred for 20 minutes, the reaction was treated with 6 mL 6 N aqueous sodium hydroxide solution, and extracted with several portions of methylene chloride. The organic phase was dried over sodium sulfate and evaporated to afford an oil, 152 mg (77%).
¹H-NMR (δ, CDCl₃): 1.28 (m, 2H), 1.48 (m, 1H), 1.72 (m, 1H), 1.96 (m, 2H), 2.22 (s, 6H), 2.37 (s, 6H), 2.78 (t, J=6, 2H), 3.67 (bs, 1H), 3.85 (bs, 1H), 4.15 (t, J=6, 2H), 5.91 (s, 2H), 6.76 (d, J=9, 1H), 7.09 (d, J=8, 1H), 7.52 (m, 2H), 7.83 (t, J=8, 1H).
¹³C-NMR (δ, CDCl₃): 13.52, 26.42, 26.66, 30.35, 39.69, 43.10, 46.13, 48.91, 58.32, 66.89, 106.68, 110.21, 118.93, 120.94, 125.55, 126.28, 127.48, 128.66, 136.12, 137.98, 148.29, 151.63, 152.78, 158.10.
MS (%): 402 (parent+1, 100).

### K. 6-[8-(N,N-Dimethylamino-ethoxy)- 1,2,3,4-tetrahydro-1,4-methano-naphthalen-5-yl]-pyridin-2-ylamine

To a 100 mL round-bottomed flask equipped with condenser and N₂ inlet were added 152 mg (0.36 mmol) 2-(2,5-dimethylpyrrolyl)-6-[8-(N,N-dimethylamino-ethoxy)-1,2,3,4-tetrahydro-1,4-methano-naphthalen-5-yl]-pyridine, 506 mg (7.6 mmol) hydroxylamine hydrochloride, 10 mL ethanol, and 1 mL water. The solution was refluxed 40 hours (LCMS P+1 = 324), cooled, poured into dilute aqueous hydrochloric acid, and washed with ethyl acetate. The aqueous layer was adjusted to pH 12 with 6 N aqueous sodium hydroxide solution and extracted with several portions of methylene chloride. The organic layer was dried over sodium sulfate and evaporated to a solid, 129 mg (78%), mp 130°C (decomposes) after conversion [how?] to the hydrochloride salt in ether.
¹H-NMR (δ, CDCl₃): 1.25 (m, 2H), 1.42 (m, 1H), 1.67 (m, 1H). 1.89 (m. 2H), 2.34 (s, 6H), 2.74 (t, J=6, 2H), 3.615 (bs, 1H), 3.74 (bs, 1H), 4.11 (t, J=6, 2H), 4.52 (bs. 2H). 6.35 (d, J=8, 1H), 6.70 (d, J=8, 1H), 6.80 (d. J=7.5, 1H), 7.36 (d, J=8, 1H), 7.42 (t, J=8, 1H).
¹³C-NMR (δ, CDCl₃): 26.5. 26.7, 39.8, 42.9, 46.1, 48.8, 58.3, 66.9, 105.9, 110.2, 113.2, 126.9, 127.5, 135.8, 137.8, 148.0, 152.2, 156.9, 158.2.
MS (%): 324 (parent+1, 100).
Analysis Calculated for C₂₀H₂₅N₃O2HCl3/2H₂O1/2(C₄H₁₀O): C 57.39, H 7.66, N 9.13. Found: C 57.58, H 7.47, N 9.09.

### EXAMPLE 2

6-[8-(2-Pyrrolidin-1-yl-ethoxy)-1,2,3,4-tetrahydro-1,4-methano-naphthalen-5-yl]-pyridin-2-ylamine Prepared as in Example 1, but using pyrrolidine instead of N,N-dimethylamine hydrochloride in step 1I above, in 45% yield as a tan solid as the hydrochloride salt from ether, mp 80°C (decomposes).
¹H-NMR (δ, CDCl₃): 1.25 (m, 2H), 1.44 (m, 1H), 1.70 (m, 1H), 1.80 (m. 4H), 1.91 (m, 2H), 2.65 (m, 4H), 2.92 (t, J=6, 2H), 3.62 (bs, 1H), 3.75 (bs, 1H). 4.16 (t. J=6, 2H), 4.46 (bs, 2H), 6.38 (d, J=8, 1H). 6.72 (d. J=8, 1H). 6.82 (d, J=7.5, 1H), 7.37 (d. J=8, 1H), 7.45 (t, J=8, 1H).
¹³C-NMR (δ, CDCl₃): 23.5, 26.4, 26.6, 39.7, 42.8, 48.7, 54.8, 55.0, 67.7, 105.8, 110.2, 113.2, 126..9, 135.7, 137.7, 147.9, 152.2, 156.9, 158.0.
MS (%): 350 (parent+1, 100).
Analysis Calculated for C₂₂H₂₇N₃O^{·}2HCl9/4H₂O 1/2(C₄H₁₀O): C 57.65, H 7.76, N 8.40. Found: C 57.65, H 7.43, N 8.34.

### EXAMPLE 3

6-[8-(2-Dimethylamino-ethoxy)-1,2,3,4-tetrahydro-1,4-ethano-naphthalen-5-yl]-pyridin-2-ylamine: Prepared as in Example 1, using as the starting material (in place of 5-Hydroxy-1,2,3,4-tetrahydro-1,4-methano-naphthalene) 5-hydroxy-1,2.3.4-tetrahydro-1,4-ethano-naphthalene, which was prepared by hydrogenation with palladium-on-carbon as catalyst followed by demethylation with boron tribromide in methylene chloride of the known (J. Med. Chem., **30**, 2191 (1987)) 5-methoxy-1,4-dihydro-1,4-ethano-naphthalene. 5-Hydroxy-1,2,3,4-tetrahydro-1,4-ethano-naphthalene gave the following ¹H-NMR data: 1.36 (m, 4H), 1.75 (m, 4H), 2.95 (singlet with fine coupling, 1H), 3.28 (singlet with fine coupling, 1H), 4.62 (bs, 1H), 6.66 (d, J=7, 1H), 6.76 (d, J=7, 1H), 7.01 (t, J=7, 1H) and mass spectral data: P=174 (60%, parent). The remaining steps were carried out as in Example 1 to give the product as a tan solid in 74% yield as the hydrochloride salt from ether, mp 130°C (dec.).
¹H-NMR (δ, CDCl₃): 1.33 (m, 4H), 1.67 (m, 4H), 2.32 (s, 6H), 2.75 (t, J=5, 2H), 3.35 (m, 1H), 3.52 (m, 1H), 4.09 (t, J=5, 2H), 4.55 (bs, 2H), 6.38 (d, J=8, 1H), 6.64 (d, J=8, 1H), 6.77 (d, J=8, 1H). 7.26 (d, J=8, 1H), 7.405 (t, J=8, 1H).
¹³C-NMR (δ, CDCl₃): 25.49, 25.72, 29.88, 45.83, 50.21, 53.37, 58.11, 63.44, 66.85, 105.96, 109.15, 114.29, 126.87, 129.90, 132.35, 137.54, 143.23, 152.76, 157.24, 158.06.
MS (%): 338 (parent+1, 100).
Analysis Calculated for C₂₁H₂₇NO^{·}2HCl3/2H₂O: C 57.66, H 7.37, N 9.61. Found: C 57.89, H 7.26, N 9.21.

### EXAMPLE 4

6-[8-(2-Pyrrolidin-1-yl-ethoxy)-1,2,3,4-tetrahydro-1,4-ethano-naphthalen-5-yl]-pyridin-2-ylamine: Prepared as in Example 3, in 87% yield as an oil as the hydrochloride salt from ether
¹H-NMR (δ, CDCl₃): 1.345 (m, 4H), 1.69 (m, 4H), 1.78 (m, 4H), 2.63 (m, 4H), 2.91 (t, J=6, 2H), 3.38 (m, 1H), 3.54 (m, 1H), 4.14 (t, J=6, 2H), 4.49 (bs, 2H). 6.38 (d. J=8, 1H). 6.67 (d, J=7, 1H). 6.79 (d. J=8. 1H). 7.27 (d, J=8, 1H), 7.41 (t, J=8, 1H).
¹³C-NMR (δ, CDCl₃): 23.44, 25.52, 25.75, 26.09, 29.88, 46.00, 54.71, 54.88, 63.40, 67.90, 105.79, 109.30, 114.30, 126.89, 129.91, 132.36, 137.45, 143.18, 152.81. 157.43, 157.99.
MS (%): 364 (parent+1, 100).
Analysis Calculated for C₂₂H₂₇N₃O2HCl 5/4H₂O: C 60.19, H 7.36, N 9.16. Found: C. 60.15. H 7.01, N 8.95.

### EXAMPLE 5

6-[8-(4-Methylpiperazin-1-yl-ethoxy)-1,2,3,4-tetrahydro-1,4-ethano-naphthalen-5-yl]-pyridin-2-ylamine: Prepared as in Example 3, in 78% yield as a tan solid as the hydrochloride salt from ether, mp 200°C (dec.).
¹H-NMR (δ, CDCl₃): 1.33 (m, 4H), 1.68 (m, 4H), 2.26 (s, 3H), 2.4-2.8 (m, 10H), 2.81 (t, J=6, 2H), 3.37 (m, 1H), 3.51 (m, 1H), 4.12 (t, J=6, 2H), 4.48 (bs, 2H), 6.38 (d, J=8, 1H), 6.66 (d, J=7, 1H), 6.77 (d, J=8, 1H), 7.26 (d, J=8, 1H), 7.41 (t, J=7, 1H).
¹³C-NMR (δ, CDCl₃): 25.59, 25.80, 26.16, 29.95, 45.88, 46.03, 53.55, 55.12, 57.17, 66.92, 105.91, 109.42, 114.40, 126.97, 130.05, 132.49, 137.55, 143.29, 152.80, 157.45, 158.07.
MS (%): 393 (parent+1, 100).
Analysis Calculated for C₂₂H₂₇N₃O^{·}3HCl3/2H₂O: C 54.50, H 7.24, N 10.59. Found: C 54.83, H 7.27, N 10.29.

### EXAMPLE 6

6-[8-(4-(2-Phenethyl)-piperazin-1-yl-ethoxy)-1,2,3,4-tetrahydro-1,4-ethano-naphthalen-5-yl]-pyridin-2-ylamine: Prepared as in Example 3, in 73.5% yield as a tan solid as the hydrochloride salt from ether, mp 130°C (dec.).
¹H-NMR (δ, CDCl₃): 1.36 (m, 4H), 1.70 (m, 4H), 1.84 (m, 2H), 2.5-2.9 (m, 12H), 3.365 (m, 1H), 3.54 (m, 1H), 4.18 (t, J=8, 2H), 4.79 (bs, 2H), 6.38 (d, J=8, 1H), 6.65 (d, J=7, 1H), 6.79 (d, J=8, 1H), 7.2-7.4 (m, 6H), 7.42 (t, J=7, 1H).
¹³C-NMR (δ. CDCl₃): 25.61, 25.83, 26.17, 30.00, 31.71, 32.59, 33.49, 53.06, 53.47, 57.17, 60.50, 66.69, 106.15, 109.25, 114.36, 126.06, 127.08, 128.40, 128.72, 129.76, 132.35, 137.70, 140.24, 143.26, 152.82, 156.33, 157.27, 158.26.
MS (%): 483 (parent+1, 100).

## Claims

1. A compound of the formula 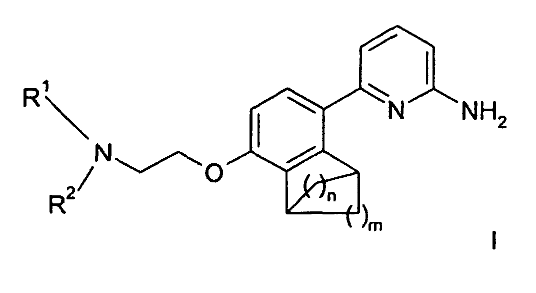 wherein n and m in the bridging rings are independently 1, 2 or 3, and a carbon in one of said bridging rings may be substituted by a heteroatom selected from O, S and N, with the proviso that a bridgehead carbon can only be substituted by nitrogen, and R¹ and R² are independently selected from C₁ to C₆ alkyl, which may be linear, branched or cyclic, and wherein each of R¹ and R² may independently be optionally substituted with from one to three substituents, preferably from zero to two substituents, that are selected, independently, from halo, nitro, hydroxy, cyano, amino, C₁ to C₄ alkoxy, and C₁ to C₄ alkylamino;
or R¹ and R² form, together with the nitrogen to which they are attached, a piperazine, azetidine, piperidine or pyrrolidine ring or an azabicyclic ring containing from 6 to 14 ring members, from 1 to 3 of which are nitrogen and the rest of which are carbon, or an azabicyclic ring of the formula 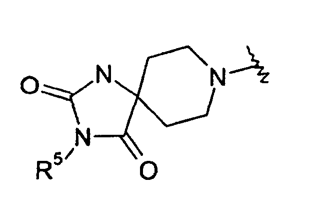 wherein R⁵ is selected from hydrogen, C₁ to C₆ alkyl, phenyl, naphthyl, phenyl-C₁ to C₆ alkyl- and naphthyl C₁ to C₆ alkyl-;
wherein the distal nitrogen on said piperazine or azabicylic ring is optionally substituted with groups R³ and R⁴ wherein R³ and R⁴ are selected from hydrogen, C₁ to C₆ alkyl, phenyl, naphthyl, C₁ to C₆ alkyl-C-(=O)-, HC(=O)-, C₁ to C₆ alkoxy-(C=O)-, phenyl-C(=O)-, naphthyl-C(=O)-, and R⁶R⁷NC(=O)- wherein R⁶ and R⁷ are selected, independently, from hydrogen and C₁ to C₆ alkyl, with the proviso that when said azabicyclic ring is a spirocyclic ring, the distal nitrogen on said spirocyclic ring is optionally substituted with R⁵ wherein R⁵ is selected from hydrogen, C₁ to C₆ alkyl, phenyl, naphthyl, phenyl-C₁ to C₆ alkyl- and naphthyl C₁ to C₆ alkyl-;
and wherein said piperazine, azetidine, piperidine and pyrrolidine rings may optionally be susbtituted with one or more substitueents, preferably with from zero to two substituents that are selected, independently, from C₁ to C₆ alkyl, amino, C₁ to C₆ alkylamino, [di-C₁-C₆ alkyl]amino, phenyl substituted 5 to 6 membered heterocyclic rings containing from 1 to 4 rings nitrogen atoms, benzoyl, benzoylmethyl, benzylcarbonyl, phenylaminocarbonyl, phenylethyl and phenoxycarbonyl, and wherein the phenyl moieties of any of the foregoing substituents may optionally be substituted with one or more substituents, preferably with from zero to two substituents, that are selected, independently, from halo, C₁ to C₃ alkyl, C₁ to C₃ alkoxy, nitro, amino, cyano, CF₃ and OCF₃;
with the proviso that no carbon atom is substituted with more than one substituent selected from hydroxy, amino, alkoxy, alkylamino and dialkylamino;
and the pharmaceutically acceptable salts of said compound.

2. A compound preferred according to claim 1, said compound selected from
6-[8-(2-Dimethylamino-ethoxy)-1,2,3,4-tetrahydro-1,4-methano-naphthalen-5-yl]-pyridin-2-ylamine;
6-[8-(2-Pyrrolidin-1-yl-ethoxy)-1,2,3,4-tetrahydro-1,4-methano-naphthalen-5-yl]-pyridin-2-ylamine;
6-[8-(2-Dimethylamino-ethoxy)-1,2,3,4-tetrahydro-1,4-ethano-naphthalen-5-yl]-pyridin-2-ylamine;
6-[8-(2-Pyrrolidin-1-yl-ethoxy)-1,2,3,4-tetrahydro-1,4-ethano-naphthalen-5-yl]-pyridin-2-ylamine.

3. A compound according to claim 1, wherein NR¹R² is an optionally substituted piperidine, azetidine, piperazine or pyrrolidine ring or a 3-aza-bicyclo[3.1.0]hex-6-ylamine ring;
and wherein said piperazine, azetidine, piperidine, pyrrolidine and 3-aza-bicyclo[3.1.0]hex-6-ylamine rings may optionally be susbtituted with one or more substituents, preferably with from zero to two substituents that are selected, independently, from C₁ to C₆ alkyl, amino, C₁ to C₆ alkylamino, [di-C₁ to C₆ alkyl]amino, phenyl, substituted 5 to 6 membered heterocyclic rings containing from 1 to 4 rings nitrogen atoms, benzoyl, benzoylmethyl, benzylcarbonyl, phenylaminocarbonyl, phenylethyl and phenoxycarbonyl, and wherein the phenyl moieties of any of the foregoing substituents may optionally be substituted with one or more substituents, preferably with from zero to two substituents, that are selected, independently, from halo, C₁ to C₃ alkyl, C₁ to C₃ alkoxy, nitro, amino, cyano, CF₃ and OCF₃.

4. A compound according to claim 1 wherein NR¹R² forms an azabicyclic ring having the formula 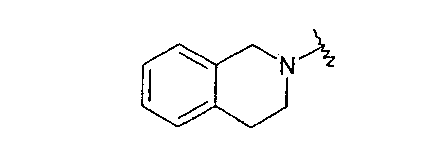 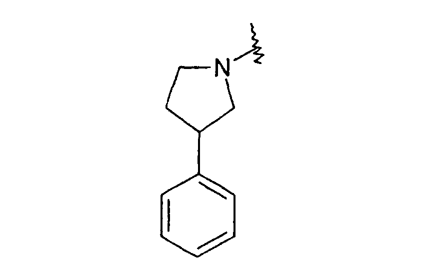 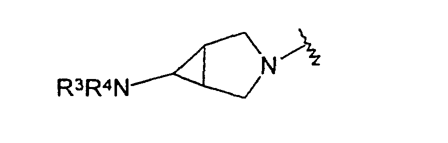 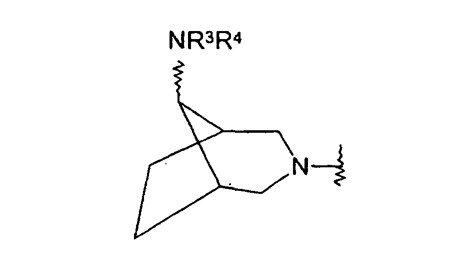 or 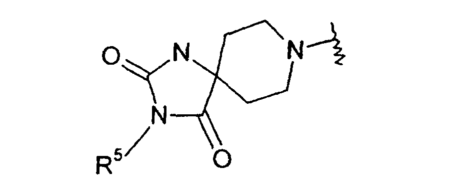 wherein R³ and R⁴ are selected from hydrogen, C₁ to C₆ alkyl, phenyl, naphthyl, C₁ to C₆ alkyl-C(=O)-, HC(=O)-, C₁ to C₆ alkoxy-(C=O)-, phenyl-C(=O)-, naphthyl-C(=O)-, and R⁶R⁷NC(=O)- wherein R⁶ and R⁷ are selected, independently, from hydrogen and C₁ to C₆ alkyl; and
R⁵ is selected from hydrogen, C₁ to C₆ alkyl, phenyl, naphthyl, phenyl-C₁ to C₆ alkyl- and naphthyl C₁ to C₆ alkyl-.

5. A pharmaceutical composition for treating or preventing a condition selected from the group consisting of migraine, inflammatory diseases, psoriasis, asthma, stroke, acute and chronic pain, hypovolemic shock, traumatic shock, reperfusion injury, Crohn's disease, ulcerative colitis, septic shock, multiple sclerosis, AIDS associated dementia, neurodegenerative diseases including Parkinson's disease, neuron toxicity, Alzheimer's disease, chemical dependencies and addictions, emesis, epilepsy, anxiety, depression, psychosis, head trauma, adult respiratory distress syndrome (ARDS), morphine induced tolerance and withdrawal symptoms, inflammatory bowel disease, osteoarthrtis, rheumatoid arthritis, ovulation, dilated cardiomyopathy, acute spinal cord injury, Huntington's disease, ocular diseases, macular degeneration, diabetic neuropathy, diabetic nephropathy and cancer in a mammal, comprising an amount of a compound according to claim 1 that is effective in treating or preventing such condition and a pharmaceutically acceptable carrier.

6. The use of a compound of the formula (I) or of a pharmaceutically acceptable salt thereof as claimed in claim 1 for the manufacture of a medicament for treating or preventing a condition selected from the group consisting of migraine inflammatory diseases, psoriasis, asthma, stroke, acute and chronic pain, hypovolemic shock, traumatic shock, reperfusion injury, Crohn's disease, ulcerative colitis, septic shock, multiple sclerosis, AIDS associated dementia, neurodegenerative diseases including Parkinson's disease, neuron toxicity, Alzheimer's disease, chemical dependencies and addictions, emesis, epilepsy, anxiety, psychosis, depression, head trauma, adult respiratory distress syndrome (ARDS), morphine induced tolerance and withdrawal symptoms, inflammatory bowel disease, osteoarthritis, rheumatoid arthritis, ovulation, dilated cardiomyopathy, acute spinal cord injury, Huntington's disease, ocular diseases diabetic neuropathy, diabetic nephropathy and cancer in a mammal.

7. A pharmaceutical composition for inhibiting nitric oxide synthase (NOS) in a mammal, according to claim 1, comprising a NOS inhibiting effective amount of a compound according to claim 1, and a pharmaceutically acceptable carrier.

8. The use of a compound of the formula (I) or pharmaceutically acceptable salt thereof as claimed in claim 1 for the manufacture of a medicament for treating or preventing a condition requiring inhibition or NOS.

9. A pharmaceutical composition for treating or preventing a condition selected from the group consisting of migraine, inflammatory diseases, psoriasis, asthma, stroke, acute and chronic pain, hypovolemic shock, traumatic shock, reperfusion injury, Crohn's disease, ulcerative colitis, septic shock, multiple sclerosis, AIDS associated dementia, neurodegenerative diseases including Parkinson's disease, neuron toxicity, Alzheimer's disease, chemical dependencies and addictions, emesis, epilepsy, anxiety, depression, psychosis, head trauma, adult respiratory distress syndrome (ARDS), morphine induced tolerance and withdrawal symptoms, inflammatory bowel disease, osteoarthritis, rheumatoid arthritis, ovulation, dilated cardiomyopathy, acute spinal cord injury, Huntington's disease, ocular diseases, macular degeneration, diabetic neuropathy, diabetic nephropathy and cancer in a mammal, comprising a NOS inhibiting effective amount of a compound according to claim 1 and a pharmaceutically acceptable carrier.

10. A compound of the formula (I) or pharmaceutically acceptable salt thereof as claimed in any one of the claims 1 to 4 for use as a medicament.

11. A compound of the formula 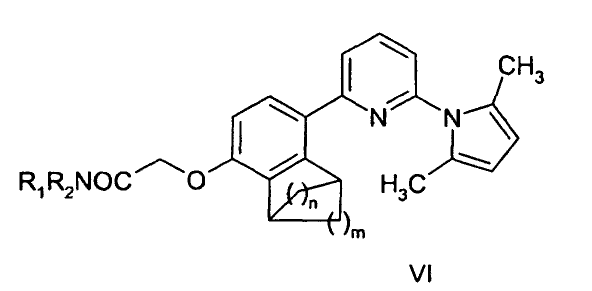 wherein m, R₁ and R₂ are as defined in claim 1.

12. A compound of the formula 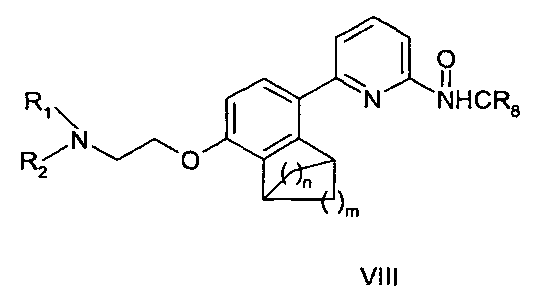 wherein n, m, R₁ and R₂ are as defined in claim 1, and R⁸ is selected from phenyl and naphthyl.

## Patentansprüche

1. Verbindung der Formel 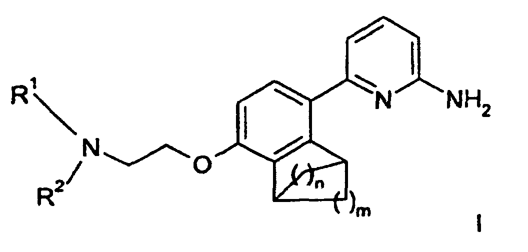 worin n und m in den Verbrückungsringen unabhängig voneinander 1, 2 oder 3 sind und ein Kohlenstoff in einem der Verbrückungsringe durch ein aus O, S und N ausgewähltes Heteroatom ersetzt sein kann, wobei ein Brückenkopf-Kohlenstoff nur durch Stickstoff ersetzt sein kann, und R¹ und R² unabhängig voneinander aus C₁-C₆-Alkyl, das linear, verzweigt oder cyclisch sein kann, ausgewählt sind und worin jeder der Reste R¹ und R² unabhängig voneinander optional mit einem bis drei Substituenten, vorzugsweise null bis zwei Substituenten, die unabhängig voneinander aus Halogen, Nitro, Hydroxy, Cyano, Amino, C₁-C₄-Alkoxy und C₁-C₄-Alkylamino ausgewählt sind, substituiert sein kann; oder R¹ und R² zusammen mit dem Stickstoff, an den sie gebunden sind, einen Piperazin-, Azetidin-, Piperidin- oder Pyrrolidinring oder einen Azabicyclischen Ring, der 6 bis 14 Ringglieder, von denen 1 bis 3 Stickstoff sind und der Rest derselben Kohlenstoff ist, enthält, oder einen Azabicyclischen Ring der Formel 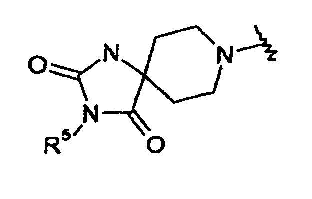 worin R⁵ aus Wasserstoff, C₁-C₆-Alkyl, Phenyl, Naphthyl, Phenyl-C₁-C₆-alkyl- und Naphthyl-C₁-C₆-alkyl- ausgewählt ist, bilden;
wobei der distale Stickstoff an dem Piperazin- oder Azabicyclischen Ring optional mit den Gruppen R³ und R⁴ substituiert ist, wobei R³ und R⁴ aus Wasserstoff, C₁-C₆-Alkyl, Phenyl, Naphthyl, C₁-C₆-Alkyl-C(=O)-, HC(=O)-, C₁-C₆-Alkoxy-(C=O)-, Phenyl-C(=O)-, Naphthyl-C(=O)- und R⁶R⁷NC(=O)-, wobei R⁶ und R⁷ unabhängig voneinander aus Wasserstoff und C₁-C₆-Alkyl ausgewählt sind, ausgewählt sind, wobei, wenn der Azabicyclischen Ring ein spirocyclischer Ring ist, der distale Stickstoff an dem spirocyclischen Ring optional mit R⁵ substituiert ist, wobei R⁵ aus Wasserstoff, C₁-C₆-Alkyl, Phenyl, Naphthyl, Phenyl-C₁-C₆-alkyl- und Naphthyl-C₁-C₆-alkyl- ausgewählt ist;
und wobei die Piperazin-, Azetidin-, Piperidin- und Pyrrolidinringe optional mit einem oder mehreren Substituenten, vorzugsweise mit null bis zwei Substituenten substituiert sein können, die unabhängig voneinander aus C₁-C₆-Alkyl, Amino, C₁-C₆-Alkylamino, (Di-C₁-C₆-alkyl)amino, Phenyl substituierten 5- bis 6-gliedrigen heterocyclischen Ringen, die 1 bis 4 Ringstickstoffatome enthalten, Benzoyl, Benzoylmethyl, Benzylcarbonyl, Phenylaminocarbonyl, Phenylethyl und Phenoxycarbonyl ausgewählt sind, und worin die Phenyleinheiten von einem beliebigen der im vorhergehenden genannten Substituenten optional mit einem oder mehreren Substituenten, vorzugsweise mit null bis zwei Substituenten substituiert sein können, die unabhängig voneinander aus Halogen, C₁-C₃-Alkyl, C₁-C₃-Alkoxy, Nitro, Amino, Cyano, CF₃ und OCF₃ ausgewählt sind;
mit dem Vorbehalt, dass kein Kohlenstoffatom mit mehr als einem Substituenten, der aus Hydroxy, Amino, Alkoxy, Alkylamino und Dialkylamino ausgewählt ist, substituiert ist;
und die pharmazeutisch akzeptablen Salze der Verbindung.

2. Bevorzugte Verbindung nach Anspruch 1, die ausgewählt ist aus
6-[8-(2-Dimethylamino-ethoxy)-1,2,3,4-tetrahydro-1,4-methano-naphthalin-5-yl]-pyridin-2-ylamin,
6-[8-(2-Pyrrolidin-1-yl-ethoxy)-1,2,3,4-tetrahydro-1,4-methano-naphthalin-5-yl]-pyridin-2-ylamin,
6-[8-(2-Dimethylamino-ethoxy)-1,2,3,4-tetrahydro-1,4-ethano-naphthalin-5-yl]-pyridin-2-ylamin,
6-[8-(2-Pyrrolidin-1-yl-ethoxy)-1,2,3,4-tetrahydro-1,4-ethano-naphthalin-5-yl]-pyridin-2-ylamin.

3. Verbindung nach Anspruch 1, worin NR¹R² ein optional substituierter Piperidin-, Azetidin-, Piperazin- oder Pyrrolidinring oder ein 3-Aza-bicyclo[3.1.0]hex-6-ylaminring ist;
und wobei die Piperazin-, Azetidin-, Piperidin-, Pyrrolidin- und 3-Aza-bicyclo[3.1.0]hex-6-ylaminringe optional mit einem oder mehreren Substituenten, vorzugsweise mit null bis zwei Substituenten substituiert sein können, die unabhängig voneinander aus C₁-C₆-Alkyl, Amino, C₁-C₆-Alkylamino, (Di-C₁-C₆-alkyl)amino, Phenyl, substituierten 5- bis 6-gliedrigen heterocyclischen Ringen, die 1 bis 4 Ringstickstoffatome enthalten, Benzoyl, Benzoylmethyl, Benzylcarbonyl, Phenylaminocarbonyl, Phenylethyl und Phenoxycarbonyl ausgewählt sind, und worin die Phenyleinheiten von einem beliebigen der im vorhergehenden genannten Substituenten optional mit einem oder mehreren Substituenten, vorzugsweise mit null bis zwei Substituenten substituiert sein können, die unabhängig voneinander aus Halogen, C₁-C₃-Alkyl, C₁-C₃-Alkoxy, Nitro, Amino, Cyano, CF₃ und OCF₃ ausgewählt sind.

4. Verbindung nach Anspruch 1, worin NR¹R² einen Azabicyclischen Ring der Formel 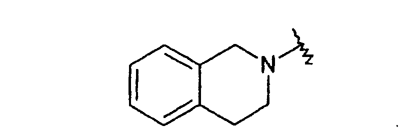 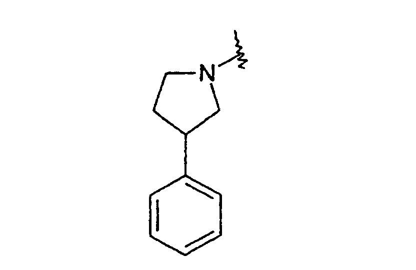 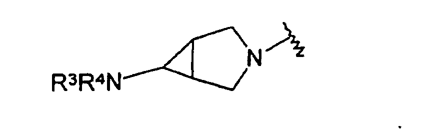 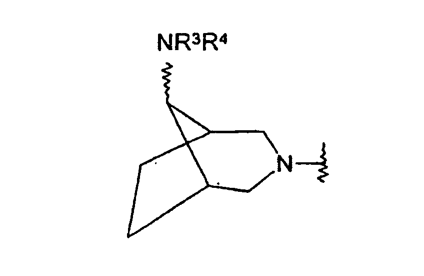 oder 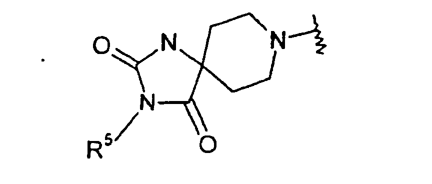 worin R³ und R⁴ aus Wasserstoff, C₁-C₆-Alkyl, Phenyl, Naphthyl, C₁-C₆-Alkyl-C(=O)-, HC(=O)-, C₁-C₆-Alkoxy-(C=O)-, Phenyl-C(=O)-, Naphthyl-C(=O)- und R⁶R⁷NC(=O)-, wobei R⁶ und R⁷ unabhängig voneinander aus Wasserstoff und C₁-C₆-Alkyl ausgewählt sind, ausgewählt sind; und R⁵ aus Wasserstoff, C₁-C₅-Alkyl, Phenyl, Naphthyl, Phenyl-C₁-C₆-alkyl- und Naphthyl-C₁-C₆-alkyl- ausgewählt ist, bildet.

5. Pharmazeutische Zusammensetzung zur Behandlung oder Prävention eines Zustands, der aus der Gruppe von Migräne, entzündlichen Erkrankungen, Psoriasis, Asthma, Apoplexie, akutem und chronischem Schmerz, hypovolämischem Schock, traumatischem Schock, einer Reperfusionsschädigung, Morbus Crohn, ulzeröser Kolitis, septischem Schock, Multipler Sklerose, mit AIDS in Verbindung stehender Demenz, neurodegenerativen Erkrankungen einschließlich Parkinson-Krankheit, Neurotoxizität, Alzheimer-Krankheit, Chemikalienabhängigkeiten und -süchten, Erbrechen, Epilepsie, Angstzuständen, Depression, einer Psychose, einem Kopftrauma, Schocklunge (ARDS), morphininduzierten Toleranz- und Entzugssymptomen, einer entzündlichen Darmerkrankung, Osteoarthritis, rheumatoider Athritis, Ovulation, dilatierter Kardiomyopathie, einer akuten Rückenmarkschädigung, Chorea Huntington, Augenerkrankungen, Makuladegeneration, diabetischer Neuropathie, diabetischer Nephropathie und Krebs ausgewählt ist, bei einem Säuger, die eine Menge einer Verbindung nach Anspruch 1, die zur Behandlung oder Prävention eines derartigen Zustands wirksam ist, und einen pharmazeutisch akzeptablen Träger umfasst.

6. Verwendung einer Verbindung der Formel (I) oder eines pharmazeutisch akzeptablen Salzes derselben gemäß Anspruch 1 zur Herstellung eines Medikaments zur Behandlung oder Prävention eines Zustands, der ausgewählt ist aus der Gruppe von Migräne, entzündlichen Erkrankungen, Psoriasis, Asthma, Schlag, akutem und chronischem Schmerz, hypovolämischem Schock, traumatischem Schock, einer Reperfusionsschädigung, Morbus Crohn, ulzeröser Kolitis, septischem Schock, Multipler Sklerose, mit AIDS in Verbindung stehender Demenz, neurodegenerativen Erkrankungen einschließlich Parkinson-Krankheit, Neurotoxizität, Alzheimer-Krankheit, Drogenabhängigkeiten und -süchten, Erbrechen, Epilepsie, Angstzuständen, einer Psychose, Depression, einem Kopftrauma, Schocklunge (ARDS), morphininduzierten Toleranz- und Entzugssymptomen, einer entzündlichen Darmerkrankung, Osteoarthritis, rheumatoider Athritis, Ovulation, dilatierter Kardiomyopathie, einer akuten Rückenmarkschädigung, Chorea Huntington, Augenerkrankungen, diabetischer Neuropathie, diabetischer Nephropathie und Krebs, bei einem Säuger.

7. Pharmazeutische Zusammensetzung zur Hemmung von Stickoxidsynthase (NOS) bei einem Säuger nach Anspruch 1, die eine zur Hemmung von NOS wirksame Menge einer Verbindung nach Anspruch 1 und einen pharmazeutisch akzeptablen Träger umfasst.

8. Verwendung einer Verbindung der Formel (I) oder eines pharmazeutisch akzeptablen Salzes derselben gemäß Anspruch 1 zur Herstellung eines Medikaments zur Behandlung oder Prävention eines Zustands, der die Hemmung von NOS erfordert.

9. Pharmazeutische Zusammensetzung zur Behandlung oder Prävention eines Zustands, der ausgewählt ist aus der Gruppe von Migräne, entzündlichen Erkrankungen, Psoriasis, Asthma, Schlag, akutem und chronischem Schmerz, hypovolämischem Schock, traumatischem Schock, einer Reperfusionsschädigung, Morbus Crohn, ulzeröser Kolitis, septischem Schock, Multipler Sklerose, mit AIDS in Verbindung stehender Demenz, neurodegenerativen Erkrankungen einschließlich Parkinson-Krankheit, Neurotoxizität, Alzheimer-Krankheit, Drogenabhängigkeiten und -süchten, Erbrechen, Epilepsie, Angstzuständen, Depression, einer Psychose, einem Kopftrauma, Schocklunge (ARDS), morphininduzierten Toleranz- und Entzugssymptomen, einer entzündlichen Darmerkrankung, Osteoarthritis, rheumatoider Athritis, Ovulation, dilatierter Kardiomyopathie, einer akuten Rückenmarkschädigung, Chorea Huntington, Augenerkrankungen, Makuladegeneration, diabetischer Neuropathie, diabetischer Nephropathie und Krebs, bei einem Säuger, die eine zur Hemmung von NOS wirksame Menge einer Verbindung nach Anspruch 1 und einen pharmazeutisch akzeptablen Träger umfasst.

10. Verbindung der Formel (I) oder ein pharmazeutisch akzeptables Salz derselben gemäß einem der Ansprüche 1 bis 4 zur Verwendung als Medikament.

11. Verbindung der Formel 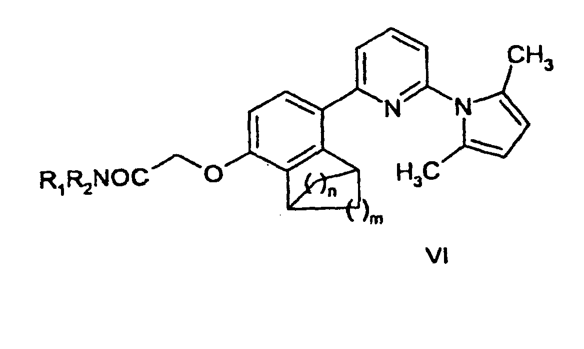 worin m, R₁ und R₂ wie in Anspruch 1 definiert sind.

12. Verbindung der Formel 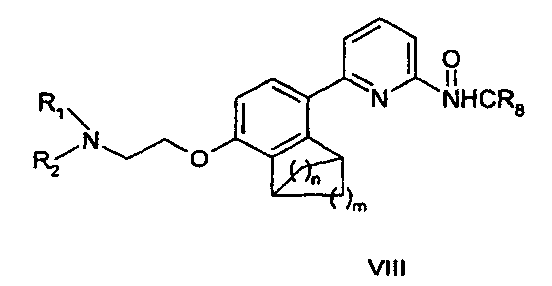 worin n, m, R₁ und R₂ wie in Anspruch 1 definiert sind und R⁸ aus Phenyl und Naphthyl ausgewählt ist.

## Revendications

1. Composé de formule 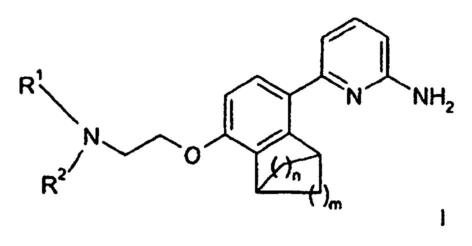 dans laquelle n et m dans les noyaux de pontage sont indépendamment égaux à 1, 2 ou 3, et un atome de carbone dans un desdits noyaux de pontage peut être substitué par un hétéroatome choisi entre O, S et N, sous réserve qu'un atome de carbone de tête de pont puisse seulement être substitué par un atome d'azote, et R¹ et R² sont choisis indépendamment parmi des groupes alkyle en C₁ à C₆, qui peuvent être linéaires, ramifiés ou cycliques, chacun des groupes R¹ et R² pouvant être indépendamment facultativement substitué avec un à trois substituants, de préférence avec zéro à deux substituants, qui sont choisis, indépendamment, entre des substituants halogéno, nitro, hydroxy, cyano, amino, alkoxy en C₁ à C₄ et alkylamino en C₁ à C₄ ;
ou bien R¹ et R² forment, conjointement avec l'atome d'azote auquel ils sont fixés, un noyau pipérazine, azétidine, pipéridine ou pyrrolidine ou un noyau azabicyclique contenant 6 à 14 membres dans le noyau, dont 1 à 3 sont des atomes d'azote et le reste consiste en atomes de carbone, ou un noyau azabicyclique de formule 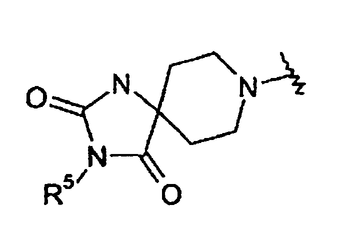 dans laquelle R⁵ est choisi entre un atome d'hydrogène, des groupes alkyle en C₁ à C₆, phényle, naphtyle, phényl-(alkyle en C₁ à C₆)- et naphtyl-(alkyle en C₁ à C₆)- ;
et dans laquelle l'atome d'azote distal sur ledit noyau pipérazine ou azabicyclique est facultativement substitué avec des groupes R³ et R⁴, les groupes R³ et R⁴ étant choisis entre un atome d'hydrogène, des groupes alkyle en C₁ à C₆, phényle, naphtyle, (alkyle en C₁ à C₆)-C-(=O)-, HC(=O), (alkoxy en C₁ à C₆)-(C=O)-, phényl-C(=O)-, naphtyl-C- (=O) - et R⁶R⁷NC(=O)- dans lesquels R⁶ et R⁷ sont choisis, indépendamment, entre un atome d'hydrogène et un groupe alkyle en C₁ à C₆, sous réserve que, lorsque ledit noyau azabicyclique est un noyau spirocyclique, l'atome d'azote distal sur ledit noyau spirocyclique soit facultativement substitué avec un substituant R⁵, R⁵ étant choisi entre un atome d'hydrogène, des groupes alkyle en C₁ à C₆, phényle, naphtyle, phényl (alkyle en C₁ à C₆)- et napthyl (alkyle en C₁ à C₆)- ;
et lesdits noyaux pipérazine, azétidine, pipéridine et pyrrolidine peuvent être facultativement substitués avec un ou plusieurs substituants, de préférence avec zéro à deux substituants, qui sont choisis, indépendamment, entre des substituants alkyle en C₁ à C₆, amino, alkylamino en C₁ à C₆, [di-(alkyle en C₁ à C₆)]amino, phényle, des noyaux hétérocycliques penta- ou hexagonaux substitués contenant un à quatre atomes d'azote dans les noyaux, benzoyle, benzoylméthyle, benzylcarbonyle, phénylamino-carbonyle, phényléthyle et phénoxycarbonyle, et les groupements phényle de n'importe lequel des substituants précités peuvent être facultativement substitués avec un ou plusieurs substituants, de préférence avec zéro à deux substituants, qui sont choisis, indépendamment, entre des substituants halogéno, alkyle en C₁ à C₃, alkoxy en C₁ à C₃, nitro, amino, cyano, CF₃ et OCF₃ ;
sous réserve qu'aucun atome de carbone ne soit substitué avec plus d'un substituant choisi entre des substituants hydroxy, amino, alkoxy, alkylamino et dialkylamino ;
et les sels pharmaceutiquement acceptables dudit composé.

2. Composé préféré suivant la revendication 1, ledit composé étant choisi entre
6-[8-(2-diméthylaminoéthoxy)-1,2,3,4-tétrahydro-1,4-méthanonaphtalène-5-yl]-pyridine-2-ylamine ;
6-[8-(2-pyrrolidine-1-yléthoxy)-1,2,3,4-tétrahydro-1,4-méthanonapthalène-5-yl]-pyridine-2-ylamine ;
6-[8-(2-diméthylaminoéthoxy)-1,2,3,4-tétrahydro-1,4-éthanonapthalène-5-yl]-pyridine-2-ylamine ;
6-[8-(2-pyrrolidine-1-yl-éthoxy)-1,2,3,4-tétrahydro-1,4-éthanonapthalène-5-yl]-pyridine-2-ylamine ;

3. Composé suivant la revendication 1, dans lequel NR¹R² représente un noyau pipéridine, azétidine, pipérazine ou pyrrolidine facultativement substitué ou un noyau 3-azabicyclo[3.1.0]hex-6-ylamine ;
et dans lequel lesdits noyaux pipérazine, azétidine, pipéridine, pyrrolidine et 3-azabicyclo[3.1.0]hex-6-ylamine peuvent être facultativement substitués avec un ou plusieurs substituants, de préférence avec 0 à 2 substituants, qui sont choisis, indépendamment, entre des substituants alkyle en C₁ à C₆, amino, alkylamino en C₁ à C₆, [di-(alkyle en C₁ à C₆)]amino, phényle, des noyaux hétérocycliques penta- ou hexagonaux substitués, contenant 1 à 4 atomes d'azote dans les noyaux, benzoyle, benzoylméthyle, benzylcarbonyle, phénylaminocarbonyle, phényléthyle et phénoxycarbonyle, et dans lequel les groupements phényle de n'importe lequel des substituants précités peut être facultativement substitué avec un ou plusieurs substituants, de préférence avec zéro à deux substituants, qui sont choisis, indépendamment, entre des substituants halogéno, alkyle en C₁ à C₃, alkoxy en C₁ à C₃, nitro, amino, cyano, CF₃ et OCF₃.

4. Composé suivant la revendication 1, dans lequel NR¹R² forme un noyau azabicyclique répondant à la formule 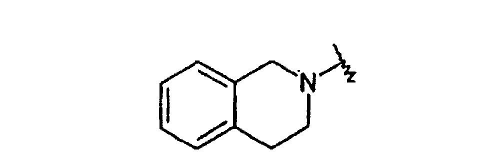 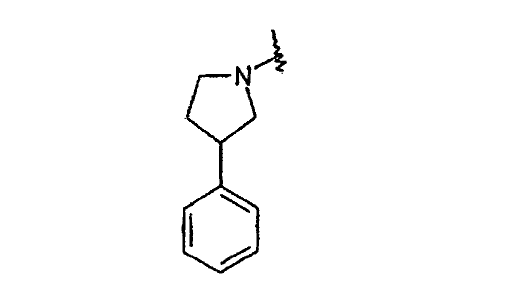 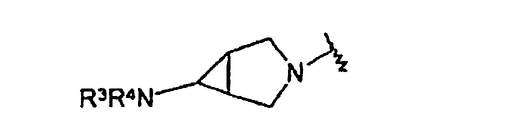 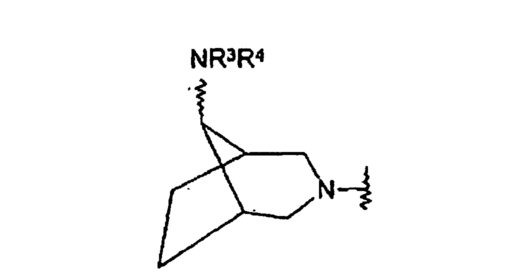 ou 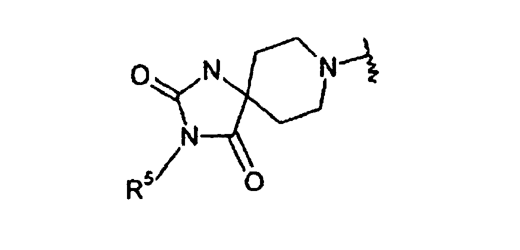 dans laquelle R³ et R⁴ sont choisis entre un atome d'hydrogène, des groupes alkyle en C₁ à C₆, phényle, napthyle, (alkyle en C₁ à C₆)-C(=O)-, HC(=O)-, (alkoxy en C₁ à C₆)-(C=O)-, phényle-C(=O)-, napthyle-C(=O)- et R⁶R⁷NC(=O)- dans lesquels R⁶ et R⁷ sont choisi, indépendamment, entre un atome d'hydrogène et un groupe alkyle en C₁ à C₆ ; et
R⁵ est choisi entre un atome d'hydrogène, des groupes alkyle en C₁ à C₆, phényle, napthyle, phényle(alkyle en C₁ à C₆)- et napthyle(alkyle en C₁ à C₆)-.

5. Composition pharmaceutique pour le traitement ou la prévention d'une affection choisie dans le groupe consistant en la migraine, des maladies inflammatoires, le psoriasis, l'asthme, un ictus, la douleur aiguë, la douleur chronique, un choc hypovolémique, un choc traumatique, une lésion de reperfusion, la maladie de Crohn, la colite ulcérative, un choc septique, la sclérose en plaques, la démence due au SIDA, des maladies neurodégénératives comprenant la maladie de Parkinson, la toxicité neuronale, la maladie d'Alzheimer, des dépendances et accoutumances chimiques, les vomissements, l'épilepsie, l'anxiété, la dépression, la psychose, un traumatisme crânien, le syndrome de détresse respiratoire de l'adulte (ARDS), les symptômes de tolérance et de sevrage induits par la morphine, une maladie intestinale inflammatoire, l'ostéoarthrite, la polyarthrite rhumatoide, l'ovulation, une myocardiopathie par dilatation, une lésion aiguë de la moelle épinière, la maladie de Huntington, des maladies oculaires, la dégénérescence maculaire, la neuropathie diabétique, la néphropathie diabétique et le cancer chez un mammifère, comprenant une quantité d'un composé suivant la revendication 1 qui est efficace dans le traitement ou la prévention d'une telle affection et un support pharmaceutiquement acceptable.

6. Utilisation d'un composé de formule (I) ou d'un de ses sels pharmaceutiquement acceptables suivant la revendication 1 pour la production d'un médicament destiné au traitement ou à la prévention d'une affection choisie dans le groupe consistant en la migraine, des maladies inflammatoires, le psoriasis, l'asthme, un ictus, la douleur aiguë, la douleur chronique, un choc hypovolémique, un choc traumatique, une lésion de reperfusion, la maladie de Crohn, la colite ulcérative, un choc septique, la sclérose en plaques, la démence due au SIDA, des maladies neurodégénératives comprenant la maladie de Parkinson, la toxicité neuronale, la maladie d'Alzheimer, des dépendances et accoutumances chimiques, les vomissements, l'épilepsie, l'anxiété, la psychose, la dépression, un traumatisme crânien, le syndrome de détresse respiratoire de l'adulte (ARDS), les symptômes de tolérance et de sevrage induits par la morphine, une maladie intestinale inflammatoire, l'ostéoarthrite, la polyarthrite rhumatoïde, l'ovulation, la myocardiopathie par dilatation, une lésion aiguë de la moelle épinière, la maladie de Huntington, des maladies oculaires, la neuropathie diabétique, la néphropathie diabétique et le cancer chez un mammifère.

7. Composition pharmaceutique pour inhiber l'oxyde nitrique-synthase (NOS) chez un mammifère, suivant la revendication 1, comprenant une quantité efficace pour inhiber la NOS, d'un composé suivant la revendication 1 et un support pharmaceutiquement acceptable.

8. Utilisation d'un composé de formule (I) ou d'un de ses pharmaceutiquement acceptables suivant la revendication 1 pour la production d'un médicament destiné au traitement ou à la prévention d'une affection nécessitant l'inhibition de la NOS.

9. Composition pharmaceutique pour le traitement ou la prévention d'une affection choisie dans le groupe consistant en la migraine, des maladies inflammatoires, le psoriasis, l'asthme, un ictus, la douleur aiguë, la douleur chronique, un choc hypovolémique, un choc traumatique, une lésion de reperfusion, la maladie de Crohn, la colite ulcérative, un choc septique, la sclérose en plaques, la démence due au SIDA, des maladies neurodégénératives comprenant la maladie de Parkinson, la toxicité neuronale, la maladie d'Alzheimer, des dépendances et accoutumances chimiques, les vomissements, l'épilepsie, l'anxiété, la dépression, la psychose, un traumatisme crânien, le syndrome de détresse respiratoire de l'adulte (ARDS), les symptômes de tolérance et de sevrage induits par la morphine, une maladie intestinale inflammatoire, l'ostéoarthrite, la polyarthrite rhumatoïde, l'ovulation, la myocardiopathie par dilatation, une lésion aiguë de la moelle épinière, la maladie de Huntington, des maladies oculaires, la dégénérescence maculaire, la neuropathie diabétique, la néphropathie diabétique et le cancer chez un mammifère, comprenant une quantité, efficace pour inhiber la NOS, d'un composé suivant la revendication 1 et un support pharmaceutiquement acceptable.

10. Composé de formule (I) ou son sel pharmaceutiquement acceptable suivant l'une quelconque des revendications 1 à 4, destiné à être utilisé comme médicament.

11. Composé de formule 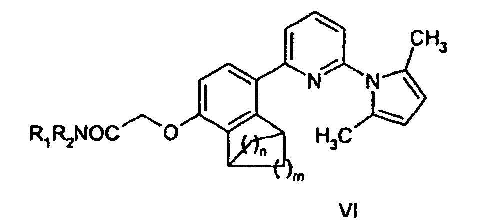 dans laquelle m, R₁ et R₂ sont tels que définis dans la revendication 1.

12. Composé de formule de formule 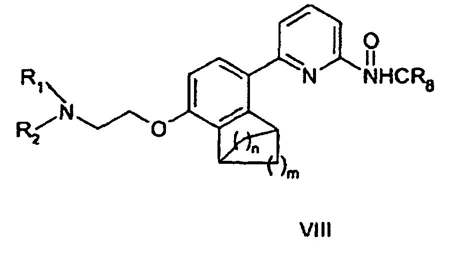 dans laquelle n, m, R₁ et R₂ sont tels que définis dans la revendication 1 et R₈ est choisi entre des groupes phényle et naphtyle.
